# EUROPEAN PATENT APPLICATION

(11) **EP 4 549 941 A1**
(43) Date of publication of application: **07.05.2025**
(21) Application number: 23830973.6
(22) Date of filing: 02.06.2023
(51) Int. Cl.: G01N 33/574, G01N 33/68

(54) **COLORECTAL CANCER BIOMARKER AND USE THEREOF**

(30) Priority: 30.06.2022 JP 2022106363
(71) Applicant: PUBLIC UNIVERSITY CORPORATION NAGOYA CITY UNIVERSITY, Nagoya-shi, Aichi 467-8601 (JP); Aichi Prefecture, Aichi 460-8501 (JP)
(72) Inventor: SHIMURA Takaya, Nagoya-shi, Aichi 467-8601 (JP); OKUDA Yusuke, Nagoya-shi, Aichi 467-8601 (JP); KATAOKA Hiromi, Nagoya-shi, Aichi 467-8601 (JP); TAGUCHI Ayumu, Nagoya-shi, Aichi 464-8681 (JP); ABE Yuichi, Nagoya-shi, Aichi 464-8681 (JP)
(74) Representative: Gramm, Lins & Partner Patent- und Rechtsanwälte PartGmbB
(86) International application number: PCT/JP2023/020674
(87) International publication number: WO 2024/004523

(57) **Abstract**

The present invention provides a colorectal cancer biomarker that enables simple and non-invasive determination of the presence or absence of the contraction of colorectal cancer with exceptional sensitivity and specificity.

The colorectal cancer biomarker of the present invention is at least one urinary protein selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.

## Description

### TECHNICAL FIELD

The present invention relates to a colorectal cancer biomarker and uses thereof.

Priority is claimed on Japanese Patent Application No. 2022-106363, filed June 30, 2022, the content of which is incorporated herein by reference.

### BACKGROUND ART

The gold standard for the diagnosis of colorectal cancer is lower gastrointestinal endoscopy and a tissue diagnosis associated with this endoscopy. However, the lower gastrointestinal endoscopy takes a long time and is a highly invasive test that causes pain to the subject during the insertion and observation of the endoscope, and the test cost is also high. In addition, there is also a risk of complications associated with the test, such as bleeding or perforation during the test, and intestinal obstruction due to laxatives for pre-treatment.

Therefore, the lower gastrointestinal endoscopy is not recommended in a screening test such as a medical checkup for healthy individuals.

Therefore, a fecal occult blood test is widely performed as a test for colorectal cancer (for example, Non Patent Documents 1 and 2). It is generally believed that according to the fecal occult blood test, the presence or absence of the contraction of colorectal cancer can be learned, and the mortality rate of colorectal cancer can be reduced. However, the fecal occult blood reaction has low detection sensitivity for early colorectal cancer. There is a report that in the test by the fecal occult blood test, the sensitivity of colorectal cancer in an advanced stage is relatively high at about 80%; however, the sensitivity of early colorectal cancer is about 28.1%.

In addition, in the fecal occult blood test, the probability that a healthy subject may be erroneously determined as having contracted colorectal cancer, that is, the probability of false positives, is extremely high. That is, the specificity of the fecal occult blood test is insufficient. This is because a positive decision in the fecal occult blood test may be due to a wound in the anus or the mucous membrane. Therefore, an endoscopy that is highly invasive and costly may be additionally performed. Moreover, the complicated handling of a specimen is still another issue with the fecal occult blood test.

From the above, there is a need for the development of a biomarker that enables a minimally invasive test. As such a biomarker, for example, carcinoembryonic antigen (CEA) and carbohydrate antigen 19-9 (CA19-9) have been proposed. However, even the detection sensitivity of these CEA and CA19-9 for colorectal cancer is low, and therefore, the use thereof in the diagnosis of colorectal cancer is not recommended.

### Citation List

### Non Patent Documents

Non Patent Document 1: National Cancer Center Japan "Cancer Information Service" (URL: https://ganjoho.jp/med_pro/pre_scr/screening/screening_colon.html, access date: June 13, 2022)
Non Patent Document 2: "Shukan Igakukai Shinbun, No. 2940, August 08, 2011", published by Igaku-Shoin, Ltd. (URL: http://www.igaku-shoin.co.jp/paperDetail.do?1d=PA02940_05, access date: June 13, 2022)

### SUMMARY OF INVENTION

### Technical Problem

The present invention provides a colorectal cancer biomarker that enables simple and non-invasive determination of the presence or absence of the contraction of colorectal cancer with exceptional sensitivity and specificity.

### Solution to Problem

According to an aspect of the present invention, there is provided a colorectal cancer biomarker which is at least one urinary protein selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.

### Advantageous Effects of Invention

According to the present invention, there is provided a colorectal cancer biomarker that enables simple and non-invasive determination of the presence or absence of the contraction of colorectal cancer with exceptional sensitivity and specificity.

### BRIEF DESCRIPTION OF DRAWINGS

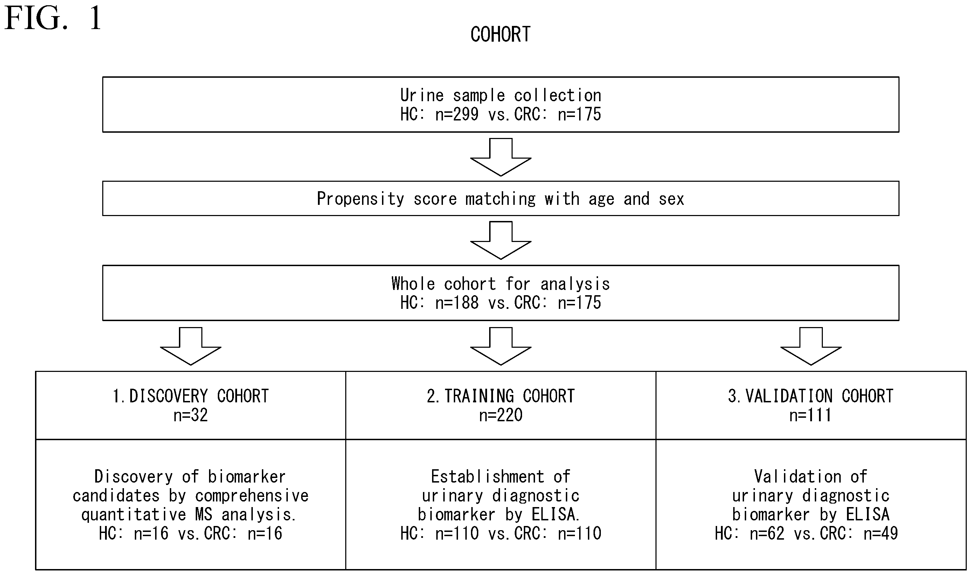

[FIG. 1] An explanatory diagram showing an outline of Examples.

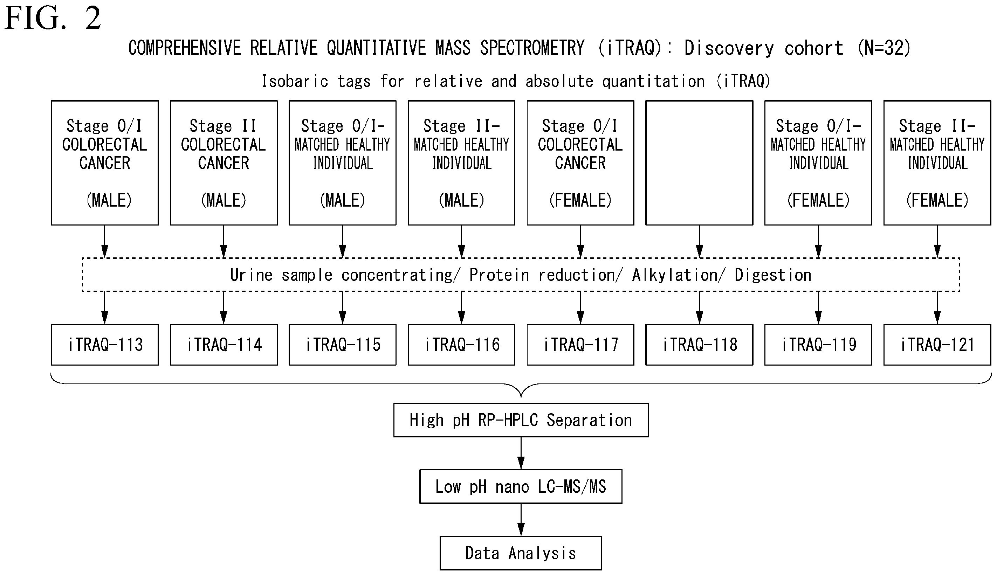

[FIG. 2] An explanatory diagram showing an overview of comprehensive relative quantitative mass spectrometry (iTRAQ (registered trademark)) using a discovery cohort.

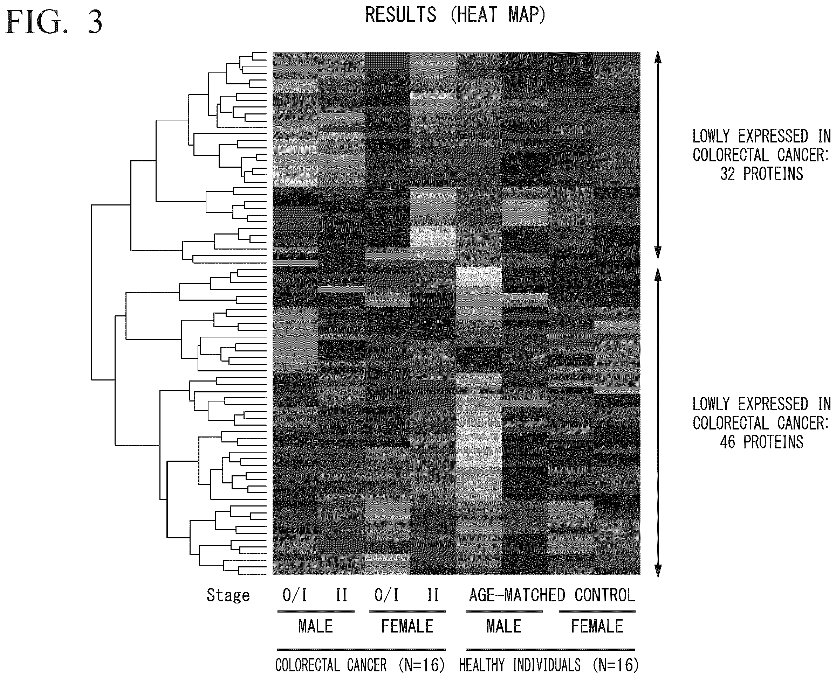

[FIG. 3] A heat map showing results of comprehensive relative quantitative mass spectrometry outlined in FIG. 2. FIG. 3 is a heat map showing proteins that are abnormally expressed in colorectal cancer patients.

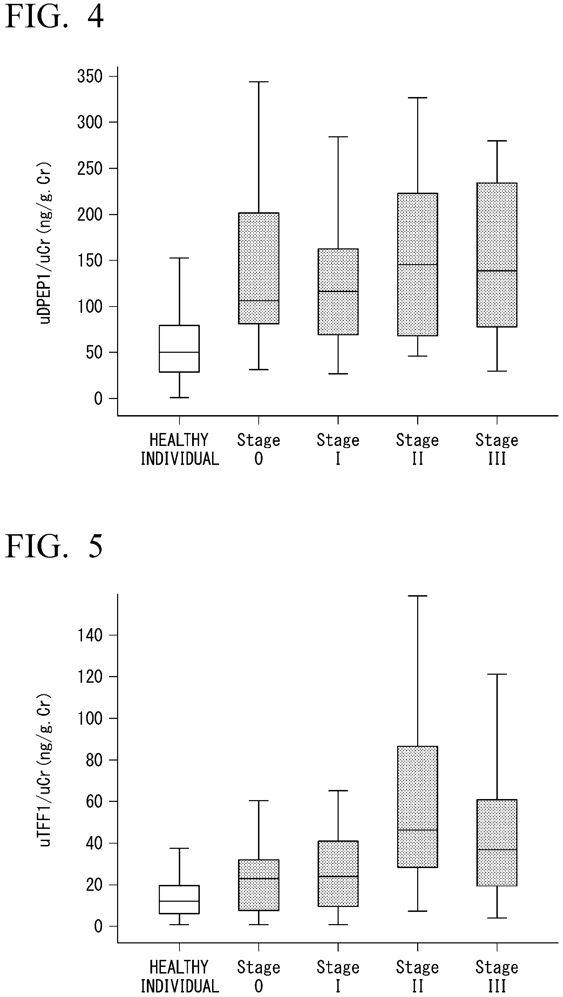

[FIG. 4] A diagram comparing the expression level of DPEP1 in urine between healthy individuals and colorectal cancer patients. For the colorectal cancer patients, the expression levels are shown in the order of the progression stage. The expression levels in the figure are values standardized by dividing the expression levels by the concentration of creatinine in urine.
[FIG. 5] A graph comparing the expression level of TFF1 in urine between healthy individuals and colorectal cancer patients. For the colorectal cancer patients, the expression levels are shown in the order of the progression stage. The expression levels in the figure are values standardized by dividing the expression levels by the concentration of creatinine in urine.

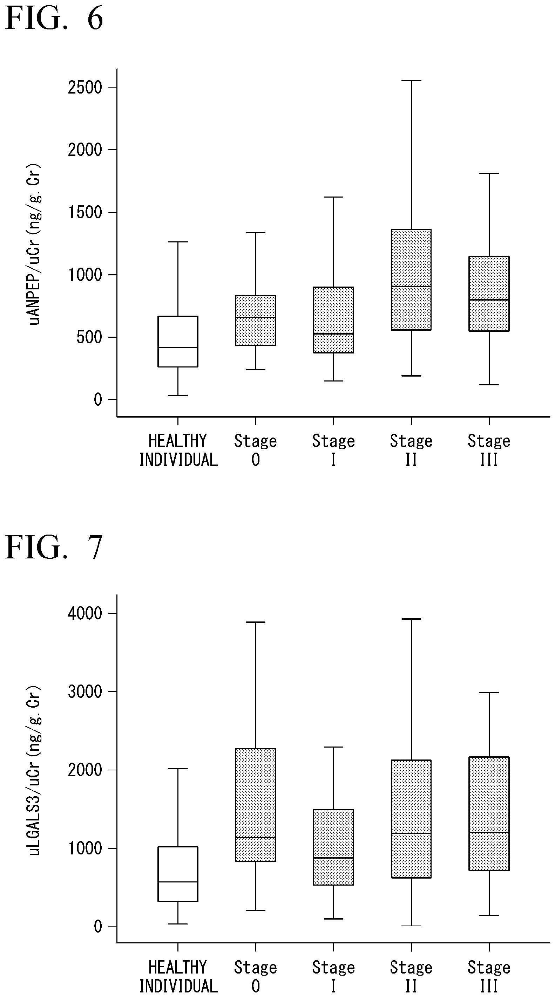

[FIG. 6] A graph comparing the expression level of ANPEP in urine between healthy individuals and colorectal cancer patients. For the colorectal cancer patients, the expression levels are shown in the order of the progression stage. The expression levels in the figure are values standardized by dividing the expression levels by the concentration of creatinine in urine.
[FIG. 7] A diagram comparing the expression level of LGALS3 in urine between healthy individuals and colorectal cancer patients. For the colorectal cancer patients, the expression levels are shown in the order of the progression stage. The expression levels in the figure are values standardized by dividing the expression levels by the concentration of creatinine in urine.

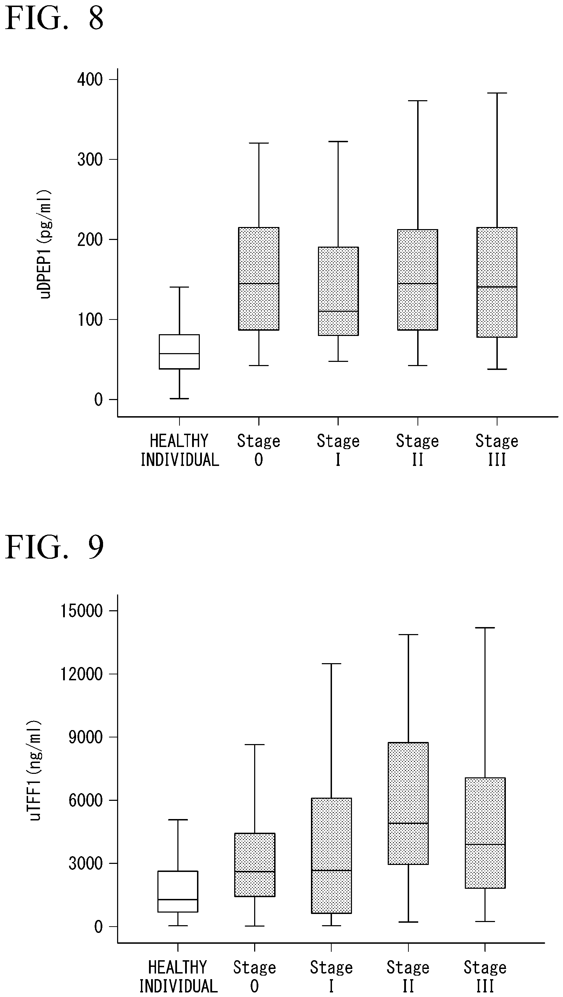

[FIG. 8] A diagram comparing the expression level of DPEP1 in urine between healthy individuals and colorectal cancer patients. For the colorectal cancer patients, the expression levels are shown in the order of the progression stage. The expression levels in the figure are absolute values before standardization.
[FIG. 9] A graph comparing the expression level of TFF1 in urine between healthy individuals and colorectal cancer patients. For the colorectal cancer patients, the expression levels are shown in the order of the progression stage. The expression levels in the figure are absolute values before standardization.

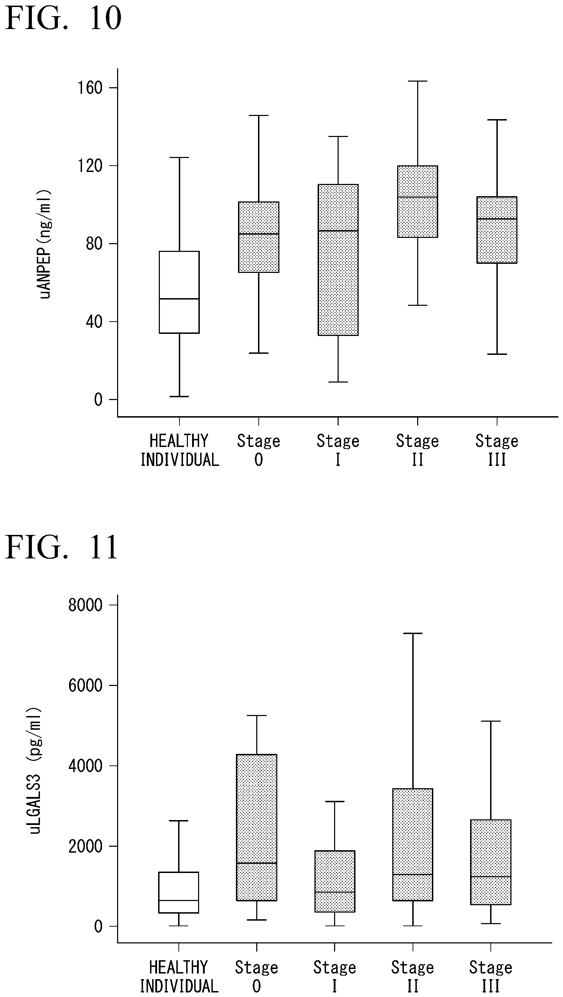

[FIG. 10] A diagram comparing the expression level of ANPEP in urine between healthy individuals and colorectal cancer patients. For the colorectal cancer patients, the expression levels are shown in the order of the progression stage. The expression levels in the figure are absolute values before standardization.
[FIG. 11] A diagram comparing the expression level of LGALS3 in urine between healthy individuals and colorectal cancer patients. For the colorectal cancer patients, the expression levels are shown in the order of the progression stage. The expression levels in the figure are absolute values before standardization.

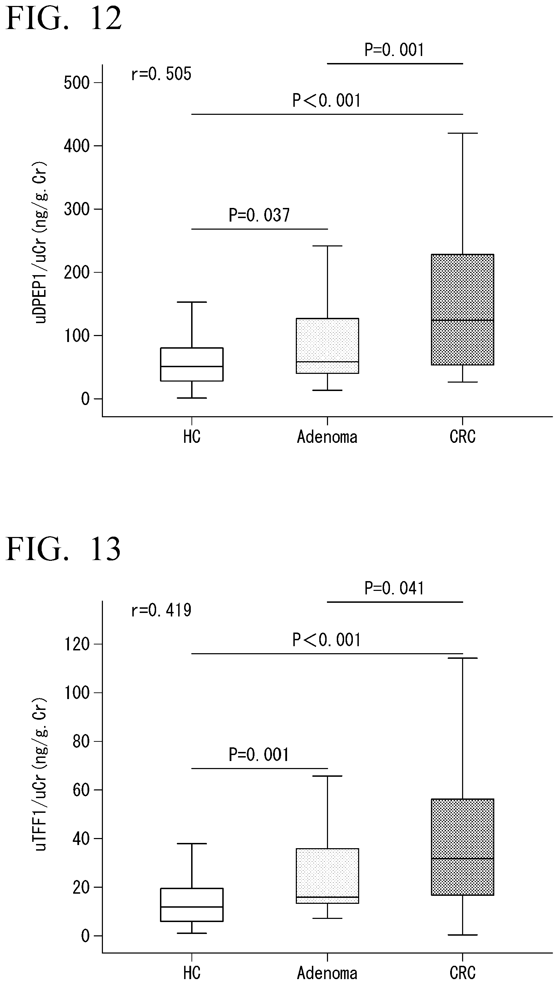

[FIG. 12] A diagram comparing the expression level of DPEP1 in urine between healthy individuals, adenoma patients, and colorectal cancer patients. The expression levels in the figure are values standardized by dividing the expression levels by the concentration of creatinine in urine.
[FIG. 13] A graph comparing the expression level of TFF1 in urine between healthy individuals, adenoma patients, and colorectal cancer patients. The expression levels in the figure are values standardized by dividing the expression levels by the concentration of creatinine in urine.

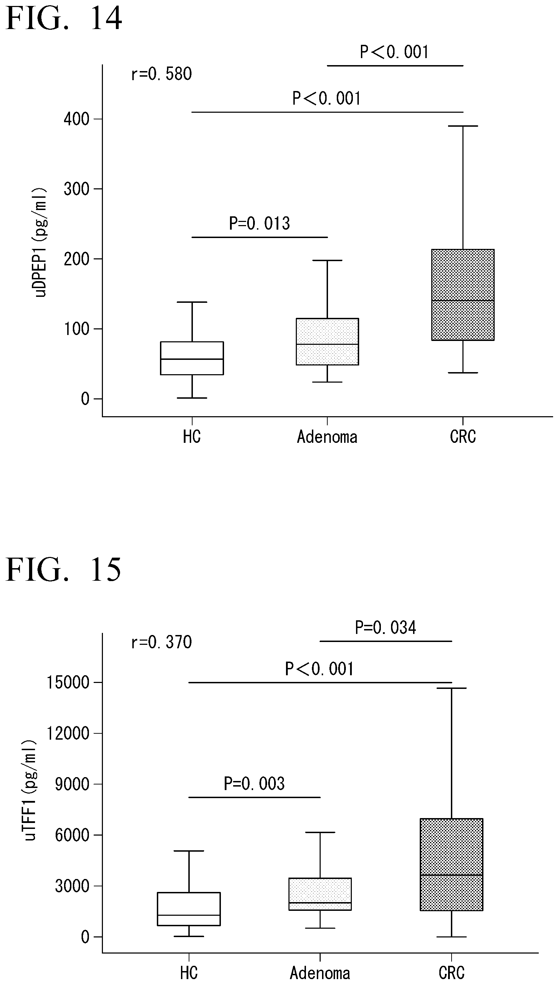

[FIG. 14] A graph comparing the expression level of DPEP1 in urine between healthy individuals, adenoma patients, and colorectal cancer patients. The expression levels in the figure are absolute values before standardization.
[FIG. 15] A graph comparing the expression level of TFF1 in urine between healthy individuals, adenoma patients, and colorectal cancer patients. The expression levels in the figure are absolute values before standardization.

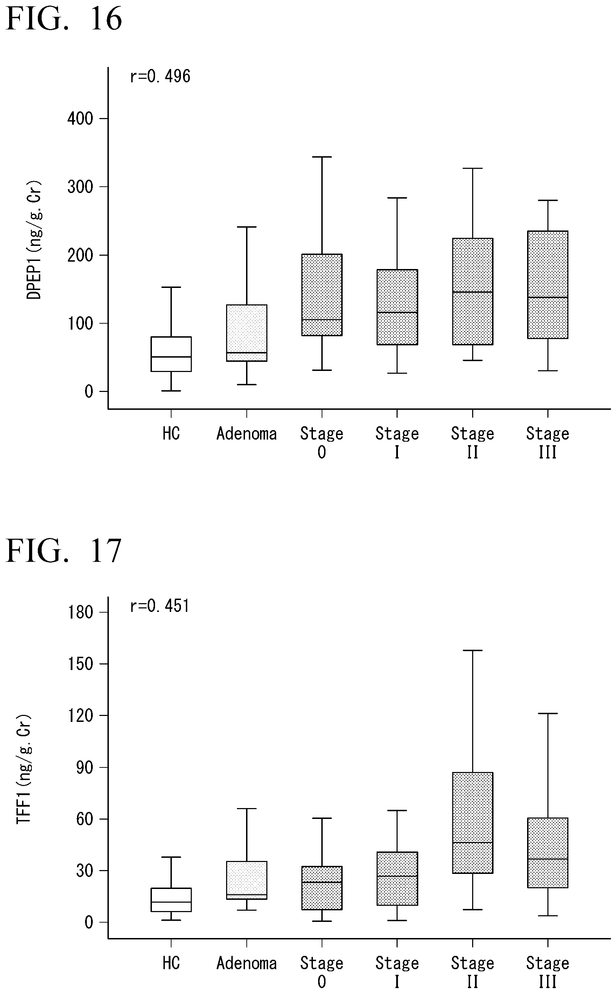

[FIG. 16] A graph comparing the expression level of DPEP1 in urine between healthy individuals, adenoma patients, and colorectal cancer patients. For the colorectal cancer patients, the expression levels are shown in the order of the progression stage. The expression levels in the figure are values standardized by dividing the expression levels by the concentration of creatinine in urine.
[FIG. 17] A diagram comparing the expression level of TFF1 in urine between healthy individuals, adenoma patients, and colorectal cancer patients. For the colorectal cancer patients, the expression levels are shown in the order of the progression stage. The expression levels in the figure are values standardized by dividing the expression levels by the concentration of creatinine in urine.

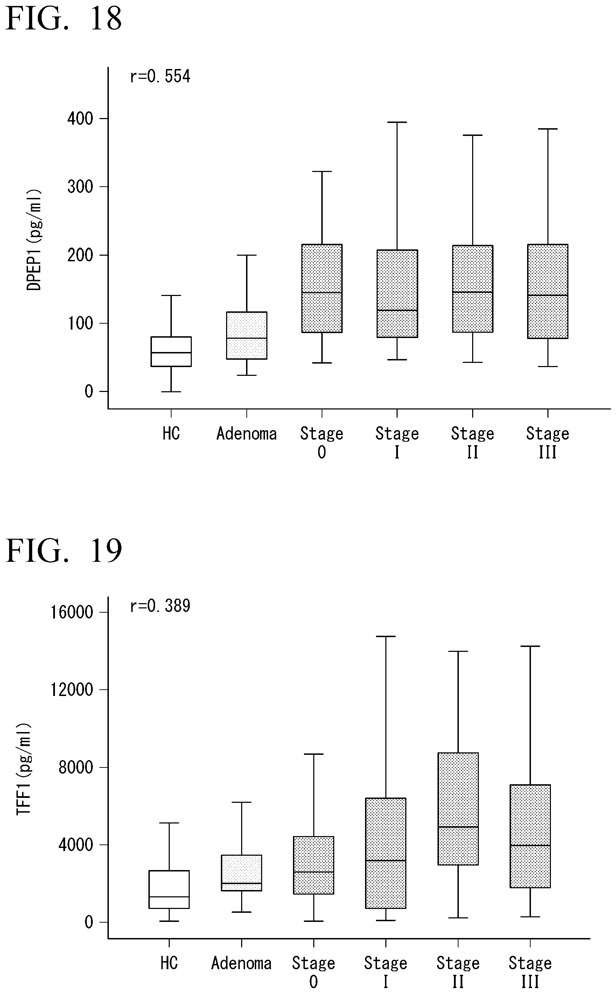

[FIG. 18] A diagram comparing the expression level of DPEP1 in urine between healthy individuals, adenoma patients, and colorectal cancer patients. For the colorectal cancer patients, the expression levels are shown in the order of the progression stage. The expression levels in the figure are absolute values before standardization.
[FIG. 19] A graph comparing the expression level of TFF1 in urine between healthy individuals, adenoma patients, and colorectal cancer patients. For the colorectal cancer patients, the expression levels are shown in the order of the progression stage. The expression levels in the figure are absolute values before standardization.

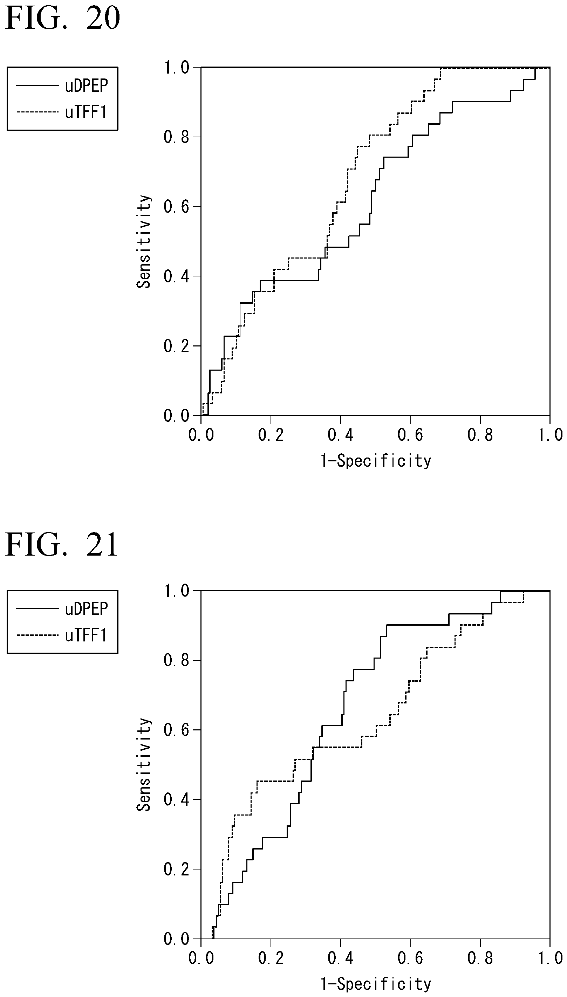

[FIG. 20] An ROC curve created to make a decision on the presence or absence of contraction of early colorectal cancer (adenoma). The expression levels are values standardized by dividing the expression levels by the concentration of creatinine in urine.
[FIG. 21] An ROC curve created to make a decision on the presence or absence of contraction of early colorectal cancer (adenoma). The expression levels are absolute values before standardization.

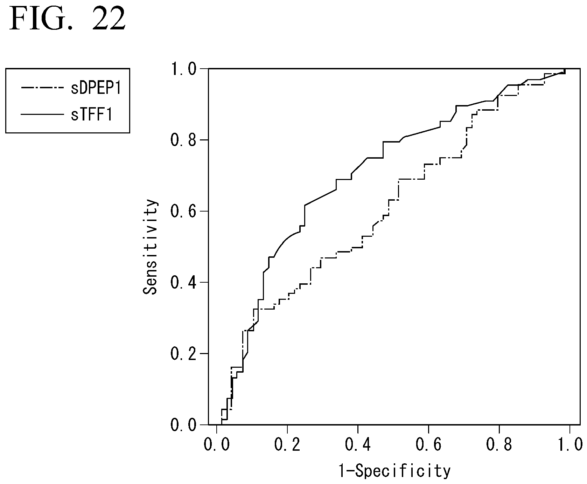

[FIG. 22] An ROC curve created based on the measured values of the protein concentration in serum specimens in order to make a decision on the presence or absence of the contraction of colorectal cancer. The expression levels are absolute values before standardization.

### DESCRIPTION OF EMBODIMENTS

In the present specification, the following terms have the following meanings.

The term "mutant" means a natural mutant caused by polymorphism, mutation, or the like, or a mutant including deletion, substitution, addition, or insertion of one or two or more bases.

The term "sensitivity" means (number of true positives)/(number of true positives + number of false negatives). Higher sensitivity makes it easier to detect colorectal cancer at an early stage, which contributes to complete resection of a cancer-affected area and a decrease in the recurrence rate. As a result, early detection can also contribute to a decrease in the mortality rate of colorectal cancer.

The term "specificity" means (number of true negatives)/(number of true negatives + number of false positives). Higher specificity makes it easier to prevent a healthy subject from being mistakenly determined to be a colorectal cancer patient, and therefore prevents conduction of unnecessary additional tests, which contributes to reducing the burden on the patients and reducing medical costs.

The term "accuracy" means (number of true positives + number of true negatives)/(total number of specimens).

The act specified by each of the terms "test", "determination", and "examination" does not include a medical practice (for example, an act of diagnosing a human illness, or an act of treating a human illness) by a physician in Japan and in countries where the medical practice is excluded from the subject of the patent.

The term "to" indicating a numerical value range means that numerical values described before and after the numerical value range are included as the lower limit value and the upper limit value.

### <Colorectal cancer biomarker>

The colorectal cancer biomarker of the present invention is at least one urinary protein selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2 (hereinafter, may be simply referred to as "urinary protein").

The present inventors have conceived to establish a protein that exhibits abnormal expression in urine of a colorectal cancer patient as a biomarker in order to simply and non-invasively test the presence or absence of the contraction of colorectal cancer.

A urine sample can be collected non-invasively. In addition, the acquisition and handling of a test sample is convenient as compared with the case of a fecal occult blood test. As described above, urine as a specimen material has many advantages for testing, such as that anyone can easily collect a specimen, that special instruments or reagents are not required for collection or storage, that a sufficient amount of a specimen can be collected at a time, that collection does not involve pain or risk, and that the circulatory dynamics in the body are reflected.

Therefore, it can be said that a urine sample is the most suitable sample for medical check-ups or screening at home.

In the present invention, a urine sample of a subject having the above-described advantages is utilized, and urinary proteins contained in very small amounts in urine and/or a combination thereof is used as a colorectal cancer biomarker. This allows the presence or absence of the contraction of colorectal cancer to be tested in a non-invasive manner.

Meanwhile, for example, various screening kits using urine specimens, such as test tapes for diabetes and a pregnancy reaction, are widely and commonly used in general clinical practice and daily life. On the other hand, since no colorectal cancer biomarker that can be detected in urine samples has been established, there is still no practical product as a screening kit for colorectal cancer utilizing a urine sample.

As described above, urine samples have exceptional advantages as a specimen for screening; however, it cannot be said that the advantages are being sufficiently utilized in the field of the colorectal cancer test.

In view of this background, according to the colorectal cancer biomarker of the present invention, it is possible to perform a test for colorectal cancer in a non-invasive and simple manner and even at home. Therefore, the colorectal cancer biomarker of the present invention is particularly useful in the field of medical check-ups or primary screening.

As shown in the Examples that will be described later, the present inventors comprehensively analyzed the protein concentrations in urine samples collected from colorectal cancer patients.

As a result of a cohort study using statistical techniques, the present inventors found that 16 specific types of urinary proteins are abnormally expressed in colorectal cancer patients.

By utilizing these urinary proteins as colorectal cancer biomarkers, the presence or absence of the contraction of colorectal cancer can be tested in a non-invasive manner.

The present inventors have identified 16 types of colorectal cancer biomarkers in urine, which are DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2. These colorectal cancer biomarkers enable determination of the presence or absence of the contraction of colorectal cancer with exceptional sensitivity and specificity. As shown in Examples that will be described later, with the colorectal cancer biomarker of the present invention, an adenoma in the large intestine can also be detected with exceptional sensitivity in addition to the early colorectal cancer.

Hereinafter, each protein will be described in order. In the following description, the "concentration" of a protein in urine can be substantially synonymous with terms such as "content", "amount present", "expression level", and "level" of the protein in urine.

DPEP1 is a polypeptide having an amino acid sequence set forth in SEQ ID NO: 1.

DPEP1 is an abbreviation for dipeptidase 1. The sequence of this protein is well known in the technical field, and can be acquired from, for example, UniProtKB-P16444. DPEP1 may be a mutant thereof.

The present inventors have found that DPEP1 is significantly highly expressed in the urine of colorectal cancer patients. According to the data acquired by the inventors, the DPEP1 concentration in urine shows a positive correlation with the contraction of colorectal cancer.

Therefore, in a case where the DPEP1 concentration in a urine sample is higher than the cut-off value, it can be determined that the subject has contracted colorectal cancer. On the other hand, in a case where the DPEP1 concentration in a urine sample is lower than the cut-off value, it can be determined that the subject has not contracted colorectal cancer.

TFF1 is a polypeptide having an amino acid sequence set forth in SEQ ID NO:2. TFF1 is an abbreviation for trefoil factor 1. The sequence of this protein is well known in the technical field, and can be acquired from, for example, UniProtKB-P04155. TFF1 may be a mutant thereof.

The present inventors have found that TFF1 is significantly highly expressed in the urine of colorectal cancer patients. According to the data acquired by the inventors, the TFF1 concentration in urine shows a positive correlation with the contraction of colorectal cancer.

Therefore, in a case where the TFF1 concentration in a urine sample is higher than the cut-off value, it can be determined that the subject has contracted colorectal cancer. On the other hand, in a case where the TFF1 concentration in a urine sample is lower than the cut-off value, it can be determined that the subject has not contracted colorectal cancer.

ANPEP is a polypeptide having an amino acid sequence set forth in SEQ ID NO:3.

ANPEP is an abbreviation for an alanyl aminopeptidase. The sequence of this protein is well known in the technical field, and can be acquired from, for example, UniProtKB-P15144. ANPEP may be a mutant thereof.

The present inventors have found that ANPEP is significantly highly expressed in the urine of colorectal cancer patients. According to the data acquired by the inventors, the ANPEP concentration in urine shows a positive correlation with the contraction of colorectal cancer.

Therefore, in a case where the ANPEP concentration in the urine sample is higher than the cut-off value, it can be determined that the subject has contracted colorectal cancer. On the other hand, in a case where the ANPEP concentration in the urine sample is lower than the cut-off value, it can be determined that the subject has not contracted colorectal cancer.

LGALS3 is a polypeptide having an amino acid sequence set forth in SEQ ID NO:4. LGALS3 is an abbreviation for galectin-3. The sequence of this protein is well known in the technical field, and can be acquired from, for example, UniProtKB-P17931. LGALS3 may be a mutant thereof.

The present inventors have found that LGALS3 is significantly highly expressed in the urine of colorectal cancer patients. According to the data acquired by the inventors, the LGALS3 concentration in urine shows a positive correlation with the contraction of colorectal cancer.

Therefore, in a case where the LGALS3 concentration in the urine sample is higher than the cut-off value, it can be determined that the subject has contracted colorectal cancer. On the other hand, in a case where the LGALS3 concentration in the urine sample is lower than the cut-off value, it can be determined that the subject has not contracted colorectal cancer.

PRG4 is a polypeptide having an amino acid sequence set forth in SEQ ID NO:5. PRG4 is an abbreviation for proteoglycan 4. The sequence of this protein is well known in the technical field, and can be acquired from, for example, UniProtKB-Q92954. PRG4 may be a mutant thereof.

The present inventors have found that PRG4 is significantly highly expressed in the urine of colorectal cancer patients. According to the data acquired by the inventors, the PRG4 concentration in urine shows a positive correlation with the contraction of colorectal cancer.

Therefore, in a case where the PRG4 concentration in a urine sample is higher than the cut-off value, it can be determined that the subject has contracted colorectal cancer. On the other hand, in a case where the PRG4 concentration in a urine sample is lower than the cut-off value, it can be determined that the subject has not contracted colorectal cancer.

CDH17 is a polypeptide having an amino acid sequence set forth in SEQ ID NO:6.

CDH17 is an abbreviation for cadherin-17. The sequence of this protein is well known in the technical field, and can be acquired from, for example, UniProtKB-Q12864. CDH17 may be a variant thereof.

The present inventors have found that CDH17 is significantly highly expressed in the urine of colorectal cancer patients. According to the data acquired by the inventors, the CDH17 concentration in urine shows a positive correlation with the contraction of colorectal cancer.

Therefore, in a case where the CDH17 concentration in a urine sample is higher than the cut-off value, it can be determined that the subject has contracted colorectal cancer. On the other hand, in a case where the CDH17 concentration in a urine sample is lower than the cut-off value, it can be determined that the subject has not contracted colorectal cancer.

PIGR is a polypeptide having an amino acid sequence set forth in SEQ ID NO:7. PIGR is an abbreviation for polymeric immunoglobulin receptor. The sequence of this protein is well known in the technical field, and can be acquired from, for example, UniProtKB-P01833. PIGR may be a mutant thereof.

The present inventors have found that PIGR is significantly highly expressed in the urine of colorectal cancer patients. According to the data acquired by the inventors, the PIGR concentration in urine shows a positive correlation with the contraction of colorectal cancer.

Therefore, in a case where the PIGR concentration in a urine sample is higher than the cut-off value, it can be determined that the subject has contracted colorectal cancer. On the other hand, in a case where the PIGR concentration in a urine sample is lower than the cut-off value, it can be determined that the subject has not contracted colorectal cancer.

TNFRSF10C is a polypeptide having an amino acid sequence set forth in SEQ ID NO:8. TNFRSF10C is an abbreviation for Tumor necrosis factor receptor superfamily member 10c. The sequence of this protein is well known in the technical field, and can be acquired from, for example, UniProtKB-O14798. The TNFRSF10C may be a variant thereof.

The present inventors have found that TNFRSF10C is significantly highly expressed in the urine of colorectal cancer patients. According to the data acquired by the inventors, the TNFRSF10C concentration in urine shows a positive correlation with the contraction of colorectal cancer.

Therefore, in a case where the TNFRSF10C concentration in a urine sample is higher than the cut-off value, it can be determined that the subject has contracted colorectal cancer. On the other hand, in a case where the TNFRSF10C concentration in a urine sample is lower than the cut-off value, it can be determined that the subject has not contracted colorectal cancer.

MEP1A is a polypeptide having an amino acid sequence set forth in SEQ ID NO:9.

MEP1A is an abbreviation for Meprin A subunit alpha. The sequence of this protein is well known in the technical field, and can be acquired from, for example, UniProtKB-Q16819. MEP1A may be a mutant thereof.

The present inventors have found that MEP1A is significantly highly expressed in the urine of colorectal cancer patients. According to the data acquired by the inventors, the MEP1A concentration in urine shows a positive correlation with the contraction of colorectal cancer.

Therefore, in a case where the MEP1A concentration in a urine sample is higher than the cut-off value, it can be determined that the subject has contracted colorectal cancer. On the other hand, in a case where the MEP1A concentration in the urine sample is lower than the cut-off value, it can be determined that the subject has not contracted colorectal cancer.

LGALS3BP is a polypeptide having an amino acid sequence set forth in SEQ ID NO:10. LGALS3BP is an abbreviation for galectin-3 binding protein. The sequence of this protein is well known in the technical field, and can be acquired from, for example, UniProtKB-Q08380. LGALS3BP may be a variant thereof.

The present inventors have found that LGALS3BP is significantly highly expressed in the urine of colorectal cancer patients. According to the data acquired by the inventors, the LGALS3BP concentration in urine shows a positive correlation with the contraction of colorectal cancer.

Therefore, in a case where the LGALS3BP concentration in the urine sample is higher than the cut-off value, it can be determined that the subject has contracted colorectal cancer. On the other hand, in a case where the LGALS3BP concentration in the urine sample is lower than the cut-off value, it can be determined that the subject has not contracted colorectal cancer.

APOA1 is a polypeptide having an amino acid sequence set forth in SEQ ID NO:11. APOA1 is an abbreviation for apolipoprotein A-1. The sequence of this protein is well known in the technical field, and can be acquired from, for example, UniProtKBP02647. APOA1 may be a variant thereof.

The present inventors have found that APOA1 is significantly highly expressed in the urine of colorectal cancer patients. According to the data acquired by the inventors, the APOA1 concentration in urine shows a positive correlation with the contraction of colorectal cancer.

Therefore, in a case where the APOA1 concentration in a urine sample is higher than the cut-off value, it can be determined that the subject has contracted colorectal cancer. On the other hand, in a case where the APOA1 concentration in the urine sample is lower than the cut-off value, it can be determined that the subject has not contracted colorectal cancer.

LRG1 is a polypeptide having an amino acid sequence set forth in SEQ ID NO:12.

LRG1 is an abbreviation for leucine rich alpha-2-glycoprotein 1. The sequence of this protein is well known in the technical field, and can be acquired from, for example, UniProtKB-P02750. LRG1 may be a mutant thereof.

The present inventors have found that LRG1 is significantly highly expressed in the urine of colorectal cancer patients. According to the data acquired by the inventors, the LRG1 concentration in urine shows a positive correlation with the contraction of colorectal cancer.

Therefore, in a case where the LRG1 concentration in a urine sample is higher than the cut-off value, it can be determined that the subject has contracted colorectal cancer. On the other hand, in a case where the LRG1 concentration in a urine sample is lower than the cut-off value, it can be determined that the subject has not contracted colorectal cancer.

ENPEP is a polypeptide having an amino acid sequence set forth in SEQ ID NO:13. ENPEP is an abbreviation for Glutamyl aminopeptidase. The sequence of this protein is well known in the technical field, and can be acquired from, for example, UniProtKB-Q07075. ENPEP may be a mutant thereof.

The present inventors have found that ENPEP is significantly highly expressed in the urine of colorectal cancer patients. According to the data acquired by the inventors, the ENPEP concentration in urine shows a positive correlation with the contraction of colorectal cancer.

Therefore, in a case where the ENPEP concentration in the urine sample is higher than the cut-off value, it can be determined that the subject has contracted colorectal cancer. On the other hand, in a case where the ENPEP concentration in the urine sample is lower than the cut-off value, it can be determined that the subject has not contracted colorectal cancer.

S100A11 is a polypeptide having an amino acid sequence set forth in SEQ ID NO:14. S100A11 is an abbreviation for Protein S100-A11. The sequence of this protein is well known in the technical field, and can be acquired from, for example, UniProtKB-P31949. S100A11 may be a mutant thereof.

The present inventors have found that S100A11 is significantly highly expressed in the urine of colorectal cancer patients. According to the data acquired by the inventors, the S100A11 concentration in urine shows a positive correlation with the contraction of colorectal cancer.

Therefore, in a case where the S100A11 concentration in a urine sample is higher than the cut-off value, it can be determined that the subject has contracted colorectal cancer. On the other hand, in a case where the S100A11 concentration in the urine sample is lower than the cut-off value, it can be determined that the subject has not contracted colorectal cancer.

MME is a polypeptide having an amino acid sequence set forth in SEQ ID NO:15. MME is an abbreviation for membrane metalloendopeptidase. The MME is also referred to as Neprilysin. The sequence of this protein is well known in the technical field, and can be acquired from, for example, UniProtKB-P08473. The MME may be a mutant thereof.

The present inventors have found that MME is significantly highly expressed in the urine of colorectal cancer patients. According to the data acquired by the inventors, the MME concentration in urine shows a positive correlation with the contraction of colorectal cancer.

Therefore, in a case where the MME concentration in a urine sample is higher than the cut-off value, it can be determined that the subject has contracted colorectal cancer. On the other hand, in a case where the MME concentration in the urine sample is lower than the cut-off value, it can be determined that the subject has not contracted colorectal cancer.

ACP2 is a polypeptide having an amino acid sequence set forth in SEQ ID NO:16.

ACP2 is an abbreviation for acid phosphatase 2. ACP2 is also referred to as lysosomal acid phosphatase. The sequence of this protein is well known in the technical field, and can be acquired from, for example, UniProtKB-P11117. ACP2 may be a variant thereof.

The present inventors have found that ACP2 is significantly lowly expressed in the urine of colorectal cancer patients. According to the data acquired by the inventors, the ACP2 concentration in urine shows a negative correlation with the contraction of colorectal cancer.

Therefore, in a case where the ACP2 concentration in the urine sample is lower than the cut-off value, it can be determined that the subject has contracted colorectal cancer. On the other hand, in a case where the ACP2 concentration in the urine sample is higher than the cut-off value, it can be determined that the subject has not contracted colorectal cancer.

As the colorectal cancer biomarker, one kind of the above-described 16 types of urinary proteins may be used alone, or two or more kinds thereof may be used in combination. Among these, the sensitivity and specificity in a case where urinary DPEP1 is used alone are extremely high, and the reproducibility is also high. However, the colorectal cancer biomarker is not limited to DPEP1. For example, at least one urinary protein selected from the group consisting of DPEP1, TFF1, ANPEP, LGALS3, PRG4, PIGR, and TNFRSF10C is also relatively useful as the colorectal cancer biomarker from the viewpoint of reproducibility.

Examples of a preferred combination of colorectal cancer biomarkers include the following (1) and (2).
· (1): Combination of DPEP1; and at least one selected from the group consisting of TFF1, ANPEP, LGALS3, PRG4, PIGR, and TNFRSF10C.
· (2): Combination of TFF1; and at least one selected from the group consisting of DPEP1, ANPEP, LGALS3, PRG4, PIGR, and TNFRSF10C.

However, the combination of the colorectal cancer biomarkers is not limited to the item (1) and the item (2). In addition to these, various combinations of urinary proteins may be useful.

Some examples of use applications of the colorectal cancer biomarkers will be described below. However, the following explanation is given as a representative example of the embodiments of the colorectal cancer biomarker of the present invention. The scope of application of the colorectal cancer biomarker of the present invention is not limited to the following description.

### <Method for testing for colorectal cancer>

A method for testing for colorectal cancer of the present invention (hereinafter, referred to as "the present method") includes detecting at least one urinary protein selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.

### (Detection of urinary protein)

The detection of a urinary protein can be carried out by a method that can be usually selected by those skilled in the art, such as an ELISA method, an immunochromatographic method, quantitative mass spectrometry, Western blotting, an immunoassay, a radioimmunoassay, a protein quantification method using a spectrophotometer, such as a BCA method or a Bradford method, or a detection method using an aptamer. Furthermore, a method of visually notifying that a urinary protein has been detected, as in a pregnancy test kit that has already been put into practical use, can also be adopted.

When detecting a urinary protein, the urinary protein concentration may be standardized by dividing the urinary protein concentration by the expression level of the protein that is considered to be constantly expressed in urine. Furthermore, an absolute value (raw data) of the urinary protein concentration may be used.

In any of the methods, colorectal cancer can be tested on the basis of the magnitude of the measured value of the protein concentration.

Examples of the protein for use in standardization, which is constantly expressed in urine, include urinary creatinine. However, from the viewpoint of simplicity, it is preferable that the amount of the colorectal cancer biomarker is used as an absolute value (raw data) without being standardized by the expression level of such a protein.

### (Cut-off value)

In an example of the present method, in a case where the urinary protein is at least one selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, and MME, when the concentration of the detected urinary protein is equal to or greater than the cut-off value, it can be determined that the subject has contracted colorectal cancer. Furthermore, in a case where the urinary protein is ACP2, when the concentration of the detected urinary protein is equal to or less than the cut-off value, it can be determined that the subject has contracted colorectal cancer.

The cut-off value can be appropriately set by those skilled in the art in consideration of sensitivity, specificity, positive diagnostic rate, positive predictive value, negative predictive value, and the like. For example, a value defined each time based on the concentration of the urinary protein in a urine sample collected from the subject who has not contracted colorectal cancer may be used as the cut-off value, or a predefined value may be used as the cut-off value.

The cut-off value is a value that can be changed depending on a measurement method and a determination method. Furthermore, the cut-off value is a value that can also be changed depending on whether improvement of sensitivity is emphasized or specificity is emphasized. The cut-off value may be set in consideration of the balance between sensitivity and specificity. Furthermore, depending on the purpose of the test, for example, in a case where the main purpose is to detect early colorectal cancer, the cut-off value may be set by prioritizing the improvement of sensitivity over specificity.

For example, as the cut-off value in a case where DPEP1 is used alone, it is believed that the relative value of urinary creatinine (Cr) is preferably 1 to 1000 ng/g.Cr, more preferably 20 to 190 ng/g.Cr, and still more preferably 35 to 128 ng/g.Cr. It is believed that the absolute value in urine is preferably 1 to 1,000 pg/ml, more preferably 35 to 130 pg/ml, and still more preferably 50 to 105 pg/ml. When the cut-off value for DPEP1 is within the above-described numerical value range, the presence or absence of the contraction of colorectal cancer can be determined with more exceptional sensitivity and specificity.

In a case of aiming at detecting early colorectal cancer of stage 0 or stage I, it is considered that the cut-off value when using DPEP1 alone is preferably 1 to 1000 ng/g.Cr, more preferably 20 to 190 ng/g.Cr, and still more preferably 35 to 130 ng/g.Cr, in terms of the urinary Cr-relative value. It is believed that the absolute value in urine is preferably 1 to 1,000 pg/ml, more preferably 35 to 130 pg/ml, and still more preferably 50 to 105 pg/ml.

In determining the presence or absence of the contraction of colorectal cancer, a method using a discriminant formula may also be used. According to the discriminant formula, colorectal cancer patients and healthy individuals can be discriminatively determined. The discriminant formula can be acquired by any discriminant analysis method. Examples of the discriminant analysis method include Fisher's discriminant analysis, nonlinear discriminant analysis using the Mahalanobis distance, a neural network, and a Support Vector Machine (SVM); however, the discriminant analysis method is not limited to these examples.

In addition, for example, determination may also be made by utilizing a k-nearest neighbor method, a decision tree, logistic regression analysis, or the like.

**In** an example of the present method, the expression level of the colorectal cancer biomarker may be compared with a reference value for the test and/or diagnosis. By comparing the expression level of the colorectal cancer biomarker with a reference value, it can be determined whether the subject has contracted colorectal cancer.

As the reference value, a reference value at which the contraction of colorectal cancer is suspected in a case where the value is equal to or greater than the reference value or in a case where the value is equal to or less than the reference value, is considered. When determining the reference value, the expression level of the colorectal cancer biomarker in a healthy subject may be referred to. Usually, the reference value is the expression level of a colorectal cancer biomarker in a healthy subject. The reference value may be set each time, or a predetermined value may be used as the reference value.

The reference value used for determination can be appropriately set according to conditions such as the age and sex of the subject, the type of colorectal cancer biomarker to be used, the collection method, and the type of specimen.

As the biological species of the subject, humans (Homo Sapience) are mainly assumed; however, the application range of the colorectal cancer biomarker of the present invention is not necessarily limited to humans. For example, when there are orthologs of each protein of the colorectal cancer biomarkers described above in various mammalian animals such as monkeys, mice, rats, dogs, cats, and rabbits, the urinary proteins of the orthologs have a possibility of being useful as colorectal cancer biomarkers in the animals as in the case of humans.

The condition of the subject is also not particularly limited. Examples of the subject include a subject for whom it is unclear whether the subject has contracted colorectal cancer; a subject for whom it has already been determined by another method that the subject has contracted colorectal cancer; a subject for whom it has already been determined that the subject has not contracted colorectal cancer; and a subject who is being treated for colorectal cancer.

**In** order to more clearly distinguish the above-described method for testing for colorectal cancer from methods for diagnosing colorectal cancer, including a doctor's judgment, it can also be said that the method for testing for colorectal cancer is synonymous with each of the following methods, that is, a method for testing for colorectal cancer, a method of collecting data for diagnosing colorectal cancer, a method for diagnosing colorectal cancer, an in vitro method for assisting in the diagnosis of colorectal cancer, and an examination method for diagnosing colorectal cancer.

According to the present invention, information that is useful for diagnosis of colorectal cancer can be provided by utilizing an objective index without relying on a doctor's judgment. In addition, a decision on the presence or absence of the contraction of colorectal cancer can be made automatically or mechanically without relying on the judgment of a person having specialized knowledge, such as a doctor or a laboratory technician.

### <Colorectal cancer test kit>

The colorectal cancer test kit is a kit used for testing for colorectal cancer.

The colorectal cancer test kit includes: a container for collecting urine of a subject; and a reagent for detecting at least one urinary protein selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.

The container is not particularly limited. Various urine collecting containers, a pipette, a cup, a centrifuge tube, and the like may be used. Furthermore, the details and preferred aspects of the colorectal cancer biomarker, that is, specific urinary proteins, are as described above.

As the reagent for detecting a urinary protein, for example, a reagent that is experimentally or practically used in techniques that can be usually selected by those skilled in the art, such as an ELISA method, an immunochromatographic method, quantitative mass spectrometry, Western blotting, an immunoassay, a radioimmunoassay, a protein quantification method using a spectrophotometer, such as a BCA method or a Bradford method, or a detection method using an aptamer, is preferred. A reagent utilizing a technique of visually notifying that a urinary protein has been detected, as in a pregnancy test kit that has already been put into practical use, may also be adopted. It is extremely useful to make the test kit simple, such as a urine pregnancy test kit, and to enable the examination at home.

In an example, the reagent for detecting a urinary protein may be either or both of the following reagent α and reagent β.

Reagent α: A reagent for notifying, by a qualitative technique, that in a case where the urinary protein is at least one selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, and MME, the subject has contracted colorectal cancer when the concentration of the detected urinary protein is equal to or greater than a threshold value.

Reagent β: A reagent for notifying, by a qualitative technique, that in a case where the urinary protein is ACP2, the subject has contracted colorectal cancer when the concentration of the detected urinary protein is equal to or less than a threshold value.

In the case of a detection reagent using a qualitative technique such as an immunochromatographic method, a threshold value for a positive test may be set. This threshold value can be appropriately set by those skilled in the art in consideration of sensitivity, specificity, positive diagnosis rate, positive predictive value, negative predictive value, and the like. For example, a predefined value based on the concentration of the urinary protein in a urine sample collected from a subject who has not contracted colorectal cancer, can be set as the threshold value.

However, the threshold value is a value that may be changed depending on whether improvement of sensitivity is emphasized or specificity is emphasized. The threshold value may also be set in consideration of the balance between sensitivity and specificity. Furthermore, depending on the purpose of the test, for example, in a case where the main purpose is to detect early colorectal cancer, the threshold value of a detection reagent using a qualitative technique such as an immunochromatographic method may be set by prioritizing sensitivity over specificity.

The colorectal cancer biomarker in the present invention targets a urinary protein. Considering the fact that existing test tapes using an immunochromatographic method or the like are commercially available, the barrier to producing a simple kit is thought to be low for those skilled in the art. Therefore, the production of a simple kit is sufficiently feasible for those skilled in the art.

The colorectal cancer test kit may be used in a facility or may be used at home. When the kit is used in a facility, the subject receives a test in the facility and collects urine in a container such as a urine sample collecting cup. The concentration of urinary proteins is measured at the facility. For the measurement of the concentration, for example, a technique that can be usually selected by those skilled in the art, such as an ELISA method, can be applied. Furthermore, even in a case of being used in a facility, the test can be carried out quickly and easily with a simple kit such as a test tape.

In a case of a kit used at home, the colorectal cancer test kit may include a urine sample container for mailing, dry ice, packaging materials, a user's manual, and the like. In a case of a simple kit, it may also be considered to purchase the kit at a drug store or the like and use the kit at home.

### <Method for treating colorectal cancer>

The method for treating colorectal cancer of the present invention includes diagnosing a subject by using the above-described colorectal cancer biomarker. In one aspect, when a subject is diagnosed to contract colorectal cancer, a therapeutic drug for colorectal cancer is administered to the subject. Furthermore, in a further aspect, when a subject is diagnosed to contract colorectal cancer, surgery is performed on the subject.

The therapeutic drug for colorectal cancer is not particularly limited. As the therapeutic drug for colorectal cancer, a drug whose potency and drug efficacy have been clinically proven is preferable. The therapeutic drug may be an anticancer agent, a molecular targeted therapeutic drug, or an immune checkpoint inhibitor.

Examples of the anticancer agent include fluorouracil, a combination drug of tegafur and uracil, a combination drug of tegafur, gimeracil, and oteracil potassium, capecitabine, irinotecan, oxaliplatin, and trifluridine-tipiracil hydrochloride.

Examples of the molecular targeted therapeutic drug include cetuximab, panitumumab, bevacizumab, ramucirumab, aflibercept, regorafenib, encorafenib, binimetinib, trastuzumab, and pertuzumab.

Examples of the immune checkpoint inhibitor include nivolumab, ipilimumab, and pembrolizumab.

However, the therapeutic drug is not limited to these examples. In a country or region other than Japan, various colorectal cancer therapeutic drugs approved in that country or region can be used.

The method of administering the drug is not particularly limited. Usually, oral administration, drip infusion, or the like is selected. However, the method of administering the drug is not limited to these examples.

The surgical procedure carried out on the subject is not particularly limited. Usually, endoscopic surgery, surgical resection, radiation therapy, or the like is selected. However, the surgical procedure carried out on the subject is not limited to these examples.

In the method for treating colorectal cancer of the present invention, since the above-described colorectal cancer biomarker is used, the subject can be treated based on diagnostic results with exceptional sensitivity and specificity. Furthermore, the detection sensitivity for early colorectal cancer is also satisfactory. Therefore, colorectal cancer can be treated at an appropriate time, which may contribute to a decrease in the mortality rate of colorectal cancer.

### <Mechanism of action>

The colorectal cancer biomarker of the present invention, which has been described above using some application examples, uses the concentration of the above-described urinary proteins and/or combinations thereof as an indicator of the presence or absence of the contraction of colorectal cancer or early colorectal cancer. Therefore, the presence or absence of the contraction of colorectal cancer can be determined with exceptional sensitivity and specificity. As shown in Examples that will be described later, with the colorectal cancer biomarker of the present invention, an adenoma in the large intestine can also be detected with exceptional sensitivity in addition to the early colorectal cancer.

In addition, since the detection of the urinary proteins is performed using urine of the subject, a non-invasive test method can be provided. Therefore, risks such as complications associated with highly invasive tests may be easily eliminated. Furthermore, the burden on the patients associated with testing can also be reduced. Then, in the handling of the urine specimen, there is an advantage that the complexity is reduced as compared with the specimen used in the fecal occult blood test.

Therefore, according to the colorectal cancer biomarker of the present invention, the presence or absence of the contraction of colorectal cancer can be determined simply and non-invasively with exceptional sensitivity and specificity.

### <Summary of embodiments>

The embodiments of the present invention include the following [1] to [14]. However, the embodiments of the present invention are not limited to the following.
[1] A method for testing for colorectal cancer, the method including:
   detecting at least one urinary protein selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.
[2] The method according to [1], further including:
   determining that, in a case where the urinary protein is at least one selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, and MME, a subject has contracted colorectal cancer when a concentration of the detected urinary protein is equal to or greater than a cut-off value; and
   determining that, in a case where the urinary protein is ACP2, a subject has contracted colorectal cancer when a concentration of the detected urinary protein is equal to or less than a cut-off value.
[3] The method according to [1] or [2], in which the urinary protein is at least one selected from the group consisting of DPEP1, TFF1, ANPEP, LGALS3, PRG4, PIGR, and TNFRSF10C.
[4] The method according to any one of [1] to [3], in which the urinary protein is a combination of: DPEP1; and at least one selected from the group consisting of TFF1, ANPEP, LGALS3, PRG4, PIGR, and TNFRSF10C.
[5] The method according to any one of [1] to [4], in which the urinary protein is a combination of: TFF1; and at least one selected from the group consisting of DPEP1, ANPEP, LGALS3, PRG4, PIGR, and TNFRSF10C.
[6] The method according to any one of [1] to [5], in which the urinary protein is at least one selected from the group consisting of DPEP1, TFF1, ANPEP, and LGALS3.
[7] The method according to any one of [1] to [6], in which the colorectal cancer is early colorectal cancer.
[8] A colorectal cancer test kit for a test of colorectal cancer, the kit including:
   a container for collecting urine of a subject; and
   a reagent for detecting at least one urinary protein selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.
[9] The colorectal cancer test kit according to [8], in which the reagent is either or both of the following reagent α and reagent β:
   reagent α: a reagent for notifying, by a qualitative technique, that in a case where the urinary protein is at least one selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, and MME, the subject has contracted colorectal cancer when the concentration of the detected urinary protein is equal to or greater than a threshold value; and
   a reagent β: a reagent for notifying, by a qualitative technique, that in a case where the urinary protein is ACP2, the subject has contracted colorectal cancer when the concentration of the detected urinary protein is equal to or less than a threshold value.
[10] The colorectal cancer test kit according to [8] or [9], in which the urinary protein is at least one selected from the group consisting of DPEP1, TFF1, ANPEP, LGALS3, PRG4, PIGR, and TNFRSF10C.
[11] The colorectal cancer test kit according to any one of [8] to [10], in which the urinary protein is a combination of: DPEP1; and at least one selected from the group consisting of TFF1, ANPEP, LGALS3, PRG4, PIGR, and TNFRSF10C.
[12] The colorectal cancer test kit according to any one of [8] to [11], in which the urinary protein is a combination of: TFF1; and at least one selected from the group consisting of DPEP1, ANPEP, LGALS3, PRG4, PIGR and TNFRSF10C.
[13] The colorectal cancer test kit according to any one of [8] to [12], in which the urinary protein is at least one selected from the group consisting of DPEP1, TFF1, ANPEP, and LGALS3.
[14] The colorectal cancer test kit according to any one of [8] to [13], in which the colorectal cancer is early colorectal cancer.

Further embodiments of the present invention include the following [15] to [23]. However, the embodiments of the present invention are not limited to the following.

[15] A method for diagnosing colorectal cancer, the method including:
detecting at least one urinary protein selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.

[16] The method according to [15], further including:
determining that, in a case where the urinary protein is at least one selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, and MME, a subject has contracted colorectal cancer when a concentration of the detected urinary protein is equal to or greater than a cut-off value; and
determining that, in a case where the urinary protein is ACP2, a subject has contracted colorectal cancer when a concentration of the detected urinary protein is equal to or less than a cut-off value.

[17] The method according to [15] or [16], in which the urinary protein is at least one selected from the group consisting of DPEP1, TFF1, ANPEP, LGALS3, PRG4, PIGR, and TNFRSF10C.

[18] The method according to any one of [15] to [17], in which the urinary protein is a combination of: DPEP1; and at least one selected from the group consisting of TFF1, ANPEP, LGALS3, PRG4, PIGR, and TNFRSF10C.

[19] The method according to any one of [15] to [18], in which the urinary protein is a combination of: TFF1; and at least one selected from the group consisting of DPEP1, ANPEP, LGALS3, PRG4, PIGR, and TNFRSF10C.

[20] The method according to any one of [15] to [19], in which the urinary protein is at least one selected from the group consisting of DPEP1, TFF1, ANPEP, and LGALS3.

[21] The method according to any one of [15] to [20], in which the colorectal cancer is early colorectal cancer.

[22] A method for treating colorectal cancer, the method including:
diagnosing a subject using the method according to any one of [15] to [21]; and
administering a therapeutic drug for colorectal cancer to the subject when the subject is diagnosed as having contracted colorectal cancer.

[23] A method for treating colorectal cancer, the method including:
diagnosing a subject using the method according to any one of [15] to [22]; and
performing surgery on the subject when the subject is diagnosed as having contracted colorectal cancer.

Further embodiments of the present invention include the following [24] to [29]. However, the embodiments of the present invention are not limited to the following.

[24] A colorectal cancer biomarker, which is at least one urinary protein selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.

[25] The colorectal cancer biomarker according to [24], in which the urinary protein is at least one selected from the group consisting of DPEP1, TFF1, ANPEP, LGALS3, PRG4, PIGR, and TNFRSF10C.

[26] The colorectal cancer biomarker according to [24] or [25], in which the urinary protein is a combination of: DPEP1; and at least one selected from the group consisting of TFF1, ANPEP, LGALS3, PRG4, PIGR, and TNFRSF10C.

[27] The colorectal cancer biomarker according to any one of [24] to [26], in which the urinary protein is a combination of: TFF1; and at least one selected from the group consisting of DPEP1, ANPEP, LGALS3, PRG4, PIGR, and TNFRSF10C.

[28] The colorectal cancer biomarker according to any one of [24] to [27], in which the urinary protein is at least one selected from the group consisting of DPEP1, TFF1, ANPEP, and LGALS3.

[29] The colorectal cancer biomarker according to any one of [24] to [28], in which the colorectal cancer is early colorectal cancer.

Further embodiments of the present invention include the following [30] to [35]. However, the embodiments of the present invention are not limited to the following.

[30] Use of a urinary protein for testing and/or diagnosis of colorectal cancer, in which the urinary protein is at least one selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.

[31] The use according to [30], in which the urinary protein is at least one selected from the group consisting of DPEP1, TFF1, ANPEP, LGALS3, PRG4, PIGR, and TNFRSF10C.

[32] The use according to [31] or [32], in which the urinary protein is a combination of: DPEP1; and at least one selected from the group consisting of TFF1, ANPEP, LGALS3, PRG4, PIGR, and TNFRSF10C.

[33] The use according to any one of [30] to [32], in which the urinary protein is a combination of: TFF1; and at least one selected from the group consisting of DPEP1, ANPEP, LGALS3, PRG4, PIGR, and TNFRSF10C.

[34] The use according to any one of [30] to [33], in which the urinary protein is at least one selected from the group consisting of DPEP1, TFF1, ANPEP, and LGALS3.

[35] The use according to any one of [30] to [34], in which the colorectal cancer is early colorectal cancer.

Although some embodiments have been described above, the present invention is not limited to the embodiments disclosed in the present specification, and can be implemented with appropriate modifications without departing from the scope of the present invention. The embodiments disclosed in the present specification can be implemented in various other forms, and various omissions, substitutions, and changes can be made without departing from the gist of the invention.

### Examples

Hereinafter, the present invention will be described in more detail with reference to Examples; however, the present invention is not limited to the following description.

### <Explanation of terms>

n: Number of cases
median: Median value
IQR: Interquartile range
P value: p value
AUC: Area Under the ROC Curve
95% CI: 95% confidence interval
HC: Healthy individual (Health Control)
CRC: Colorectal cancer patient
Univariate analysis: Univariate analysis
Multivariate analysis: Multivariate analysis

In the tables and drawings shown below, the prefix "u" of each protein means that the protein is a urinary protein.

### <Construction of cohort>

The following three types of random age- and sex-matched cohorts were constructed from urine specimens collected from 175 age- and sex-matched colorectal cancer patients in clinical stage 0, stage I, stage II, and stage III and 299 healthy individuals.
· Discovery cohort: 32 cases consisting of 16 cases of colorectal cancer patients and 16 cases of healthy individuals.
· Training cohort: 220 cases consisting of 110 cases of colorectal cancer patients and 110 cases of healthy individuals.
· Validation cohort: 111 cases consisting of 62 cases of colorectal cancer patients and 49 cases of healthy individuals.

### <Narrowing down of biomarker candidate>

In the present invention, relative quantitative mass spectrometry was used when narrowing down the biomarkers. Furthermore, when establishing and validating the biomarkers, the concentration of each urinary protein was measured using an ELISA method that utilized an antigen-antibody reaction.

The discovery cohort was used for discovering a biomarker candidate. Comprehensive analysis using relative quantitative mass spectrometry (iTRAQ (registered trademark)) was performed on the 32 cases of the discovery cohort (comprehensive analysis cohort). The concentration of the urine samples, and the reduction reaction, alkylation, and digestion reaction of proteins were carried out according to general techniques. Subsequently, as shown in FIG. 2, High pH RP-HPLC Separation and Low pH nano LC-MS/MS were performed in this order, and data analysis was carried out.

As a result, 78 types of urinary proteins that were significantly abnormally expressed in the urine of the colorectal cancer cases were identified (FIG. 3). Among these, 23 types of proteins satisfying gastrointestinal tract specificity and tumor specificity were narrowed down as biomarker candidates by database search.

### <Establishment of colorectal cancer biomarker>

The training cohort was used for establishing colorectal cancer biomarkers. The concentrations of the 23 types of urinary proteins as biomarker candidates narrowed down by the analysis of the discovery cohort were measured by ELISA for the urine specimens of the 220 cases of the training cohort.

For the convenience of analysis, the urinary protein concentration was acquired not only as an absolute value, which was the measured value itself, but also as a urinary Cr-relative value standardized by dividing the urinary protein concentration by the urinary creatinine concentration. The urinary creatinine is usually a protein that is widely expressed in urine of a specimen. The candidate protein concentration was standardized by standardizing the protein concentration of a biomarker candidate using the urinary creatinine concentration. The results are shown in Table 1 to Table 4.

In the data analysis results shown below, AUC is a statistical index and takes a value from 0 to 1. As the value of AUC is closer to 1, it indicates that the higher the determination ability of the presence or absence of the contraction of colorectal cancer, that is, the diagnostic ability, is higher.

**[Table 1]**

| Training cohort (n = 220) | Univariate analysis | | | AUC [95% CI] | Multivariate analysis | |
|---|---|---|---|---|---|---|
| | HC Median [IQR] | CRC Median [IQR] | P value | | Odds ratio [95% CI] | P value |
| uDPEP1/uCr (ng/g.Cr) | 49.3 | 118.6 | < 0.001 | 0.802 | 1.010 | 0.004 |
| | [30.5 to 78.6] | [77.7 to 193.9] | | [0.745 to 0.860] | [1.003 to 1.016] | |
| uTFF1/uCr (ng/g.Cr) | 12.4 | 34.7 | < 0.001 | 0.751 | 1.027 | < 0.001 |
| | [7.0 to 19.3] | [16.8 to 58.0] | | [0.685 to 0.817] | [1.014 to 1.041] | |
| uANPEP/uCr (ng/g.Cr) | 415.9 | 714.8 | < 0.001 | 0.714 | 0.999 | 0.185 |
| | [269.2 to 669.5] | [488.3 to 1061.2] | | [0.645 to 0.782] | [0.998 to 1.000] | |
| uLGALS3/uCr (ng/g.Cr) | 563.9 | 1132.1 | < 0.001 | 0.712 | 1.000 | 0.179 |
| | [306.3 to 1012.9] | [592.6 to 2276.7] | | [0.644 to 0.780] | [1.000 to 1.001] | |
| uPRG4/uCr (ng/g.Cr) | 93.7 | 164.0 | < 0.001 | 0.709 | 1.003 | 0.087 |
| | [61.9 to 139.4] | [87.9 to 329.8] | | [0.640 to 0.777] | [1.000 to 1.006] | |
| uCDHI17/uCr (ng/g.Cr) | 361.8 | 624.0 | < 0.001 | 0.701 | 1.000 | 0.064 |
| | [230.1 to 538.6] | [363.8 to 1277.7] | | [0.632 to 0.770] | [0.999 to 1.000] | |
| uPIGR/uCr (ng/g.Cr) | 159.0 | 271.8 | < 0.001 | 0.688 | 1.002 | 0.157 |
| | [109.8 to 230.1] | [141.2 to 463.5] | | [0.615 to 0.760] | [0.999 to 1.004] | |
| uTNFRSF10C/uCr (µg/g.Cr) | 8.2 | 10.1 | 0.002 | 0.622 | 0.960 | 0.308 |
| | [6.5 to 11.0] | [7.7 to 13.0] | | [0.548 to 0.696] | [0.887 to 1.039] | |
| uMME/uCr (ng/g.Cr) | 33.4 | 54.9 | < 0.001 | 0.660 | - | - |
| | [19.7 to 57.0] | [27.9 to 93.2] | | [0.572 to 0.747] | - | - |
| uLRG1/uCr (ng/g.Cr) | 116.4 | 168.2 | 0.001 | 0.634 | - | - |
| | [45.6 to 189.3] | [93.4 to 306.9] | | [0.545 to 0.723] | - | - |
| uLGALS3BP/uCr (µg/g.Cr) | 1.2 | 2.0 | 0.001 | 0.623 | - | - |
| | [0.6 to 2.2] | [1.0 to 3.1] | | [0.534 to 0.713] | - | - |

**[Table 2]**

| Training cohort (n = 220) | Univariate analysis | | | AUC [95% CI] | Multivariate analysis | |
|---|---|---|---|---|---|---|
| | HC Median [IQR] | CRC Median [IQR] | P value | | Odds ratio [95% CI] | P value |
| uS100A11/uCr (ng/g.Cr) | 585.5 | 928.6 | 0.008 | 0.623 | - | - |
| | [275.5 to 1276.6] | [445.5 to 1906.5] | | [0.534 to 0.713] | - | - |
| uAPOA1/uCr (ng/g.Cr) | 50.9 | 82.7 | 0.012 | 0.617 | - | - |
| | [15.7 to 113.7] | [27.0 to 264.2] | | [0.527 to 0.707] | - | - |
| uENPEP/uCr (ng/g.Cr) | 137.3 | 216.1 | 0.032 | 0.595 | - | - |
| | [96.6 to 228.0] | [101.3 to 340.6] | | [0.503 to 0.688] | - | - |
| uMEP1A/uCr (ng/g.Cr) | 71.5 | 92.2 | 0.032 | 0.571 | - | - |
| | [57.7 to 99.2] | [53.5 to 148.7] | | [0.475 to 0.666] | - | - |
| uCTSC/uCr (ng/g.Cr) | 147.3 | 248.7 | 0.072 | 0.579 | - | - |
| | [97.3 to 252.8] | [103.2 to 356.8] | | [0.486 to 0.673] | - | - |
| uGC/uCr (ng/g.Cr) | 246.2 | 210.7 | 0.258 | 0.461 | - | - |
| | [150.3 to 409.1] | [91.3 to 452.1] | | [0.366 to 0.555] | - | - |
| uACP2/uCr (µg/g.Cr) | 2.1 | 2.6 | 0.287 | 0.538 | - | - |
| | [1.7 to 3.1] | [1.5 to 3.7] | | [0.443 to 0.633] | - | - |
| uDDAH1/uCr (ng/g.Cr) | 683.4 | 791.4 | 0.564 | 0.531 | - | - |
| | [264.5 to 1357.2] | [260.3 to 1903.4] | | [0.437 to 0.624] | - | - |
| uSPP1/uCr (µg/g.Cr) | 999.5 | 834.2 | 0.230 | 0.419 | - | - |
| | [692.8 to 1263.0] | [392.2 to 1150.1] | | [0.286 to 0.552] | - | - |
| uANXA7/uCr (ng/g.Cr) | 219.1 | 311.1 | 0.245 | 0.604 | - | - |
| | [153.7 to 382.9] | [174.5 to 445.5] | | [0.475 to 0.732] | - | - |
| uORM1/uCr (µg/g.Cr) | 186.4 | 191.5 | 0.683 | 0.526 | - | - |
| | [138.8 to 286.4] | [122.4 to 383.5] | | [0.391 to 0.661] | - | - |
| uORM2/uCr (mg/g.Cr) | 1.8 | 3.2 | 0.187 | 0.574 | - | - |
| | [0.6 to 4.4] | [0.5 to 7.8] | | [0.480 to 0.668] | - | - |

**[Table 3]**

| Training cohort (n = 220) | Univariate analysis | | | AUC [95% CI] | Multivariate analysis | |
|---|---|---|---|---|---|---|
| | HC Median [IQR] | CRC Median [IQR] | P value | | Odds ratio [95% CI] | P value |
| uDPEP1 (pg/ml) | 65.1 | 105.9 | < 0.001 | 0.799 | 1.015 | < 0.001 |
| | [42.9 to 81.9] | [74.2 to 176.3] | | [0.741 to 0.857] | [1.007 to 1.023] | |
| uTFF1 (ng/ml) | 1.4 | 3.5 | < 0.001 | 0.697 | 1.000 | 0.011 |
| | [0.8 to 2.7] | [1.4 to 7.1] | | [0.626 to 0.768] | [1.000 to 1.000] | |
| uANPEP (ng/ml) | 53.7 | 89.7 | < 0.001 | 0.698 | 1.010 | 0.072 |
| | [34.4 to 76.9] | [53.7 to 105.8] | | [0.627 to 0.769] | [0.999 to 1.022] | |
| uLGALS3 (pg/ml) | 641.8 | 1036.4 | 0.001 | 0.623 | 1.000 | 0.889 |
| | [330.8 to 1382.4] | [486.8 to 2773.7] | | [0.549 to 0.698] | [1.000 to 1.000] | |
| uPRG4 (pg/ml) | 101.8 | 150.4 | < 0.001 | 0.674 | 1.005 | 0.030 |
| | [82.4 to 148.7] | [100.4 to 268.6] | | [0.603 to 0.745] | [1.000 to 1.009] | |
| uCDH17 (pg/ml) | 444.2 | 633.8 | < 0.001 | 0.712 | 1.000 | 0.908 |
| | [340.7 to 555.7] | [444.2 to 1155.1] | | [0.643 to 0.781] | [1.000 to 1.000] | |
| uPIGR (ng/ml) | 18.3 | 23.9 | < 0.001 | 0.636 | 1.000 | 0.268 |
| | [14.0 to 24.9] | [15.9 to 42.8] | | [0.561 to 0.710] | [1.000 to 1.000] | |
| uTNFRSF10C (ng/ml) | 10.5 | 10.4 | 0.809 | 0.506 | 0.904 | 0.002 |
| | [6.5 to 15.3] | [6.0 to 17.6] | | [0.428 to 0.584] | [0.848 to 0.963] | |
| uMME (pg/ml) | 40.2 | 50.8 | 0.003 | 0.634 | - | - |
| | [25.4 to 59.3] | [33.9 to 67.7] | | [0.545 to 0.723] | | |
| uLRG1 (ng/ml) | 16.1 | 24.4 | < 0.001 | 0.682 | - | - |
| | [6.8 to 23.3] | [13.1 to 25.5] | | [0.595 to 0.769] | | |
| uLGALS3BP (ng/ml) | 133.0 | 176.8 | 0.084 | 0.572 | - | - |
| | [86.0 to 251.9] | [96.1 to 285.1] | | [0.480 to 0.665] | | |
| uS100A11 (ng/ml) | 0.6 | 1.1 | 0.027 | 0.609 | - | - |
| | [0.3 to 1.3] | [0.5 to 1.8] | | [0.517 to 0.700] | | |

**[Table 4]**

| Training cohort (n = 220) | Univariate analysis | | | AUC [95% CI] | Multivariate analysis | |
|---|---|---|---|---|---|---|
| | HC Median [IQR] | CRC Median [IQR] | P value | | Odds ratio [95% CI] | P value |
| uAPOA1 (ng/ml) | 5.5 | 7.8 | 0.097 | 0.588 | - | - |
| | [2.2 to 13.9] | [3.7 to 24.1] | | [0.496 to 0.680] | | |
| uENPEP (pg/ml) | 178.1 | 196.4 | 0.459 | 0.550 | - | - |
| | [145.4 to 225.4] | [142.1 to 254.8] | | [0.456 to 0.643] | | |
| uMEP1A (ng/ml) | 9.5 | 9.3 | 0.469 | 0.481 | - | - |
| | [8.5 to 10.3] | [8.3 to 10.3] | | [0.387 to 0.574] | | |
| uCTSC (pg/ml) | 178.1 | 201.5 | 0.362 | 0.555 | - | - |
| | [143.4 to 236.3] | [154.7 to 273.2] | | [0.462 to 0.648] | | |
| uGC (ng/ml) | 36.6 | 24.4 | 0.052 | 0.420 | - | - |
| | [18.9 to 59.7] | [6.3 to 63.6] | | [0.327 to 0.513] | | |
| uACP2 (ng/ml) | 2.7 | 2.5 | < 0.001 | 0.650 | - | - |
| | [2.4 to 2.9] | [2.1 to 2.8] | | [0.562 to 0.739] | | |
| uDDAH1 (pg/ml) | 863.6 | 607.0 | 0.514 | 0.479 | - | - |
| | [363.4 to 1662.7] | [254.8 to 2998.1] | | [0.384 to 0.574] | | |
| uSPP1 (ng/ml) | 1223.2 | 716.5 | 0.104 | 0.391 | - | - |
| | [535.4 to 1805.1] | [360.2 to 1476.5] | | [0.260 to 0.522] | | |
| uANXA7 (ng/ml) | 310.8 | 324.3 | 0.779 | 0.544 | - | - |
| | [223.0 to 398.7] | [202.7 to 446.0] | | [0.411 to 0.676] | | |
| uORM1 (ng/ml) | 247.5 | 248.5 | 0.116 | 0.601 | - | - |
| | [243.3 to 248.8] | [238.8 to 250.1] | | [0.466 to 0.736] | | |
| uORM2 (µg/ml) | 2.2 | 2.8 | 0.458 | 0.542 | - | - |
| | [0.7 to 5.2] | [0.5 to 10.8] | | [0.448 to 0.637] | | |

From the analysis results of the training cohort, 15 types of colorectal cancer biomarkers, that is, DPEP1, TFF1, ANPEP, LGALS3, PRG4, CDH17, PIGR, TNFRSF10C, MEP1A, LGALS3BP, APOA1, LRG1, ENPEP, S100A11, and MME were established from the 23 types of biomarker candidates based on the analysis results with the urinary Cr-relative values (Table 1 and Table 2).

In addition, in the analysis results with the absolute value, 11 types of colorectal cancer biomarkers, that is, DPEP1, TFF1, ANPEP, LGALS3, PRG4, CDH17, PIGR, LRG1, ACP2, S100A11, and MME were established (Table 3 and Table 4).

As shown in Table 1 to Table 4, these identified colorectal cancer biomarkers have p-values lower than those of the proteins of the other biomarker candidates. From this, it can be seen that the colorectal cancer biomarkers are significantly abnormally expressed in the urine of colorectal cancer patients.

Among these, 10 types of DPEP1, TFF1, ANPEP, LGALS3, PRG4, CDH17, PIGR, LRG1, S100A11, and MME have low p values regardless of the presence or absence of standardization based on urinary Cr, and therefore, the reproducibility is high.

### <Verification of colorectal cancer biomarker>

Next, 111 cases of validation cohort independent from both the discovery cohort and the training cohort were used. The validation cohort was used for verification of the colorectal cancer biomarkers established through the discovery cohort and the training cohort.

In this verification, the diagnostic accuracy for colorectal cancer was verified for 8 types of urinary proteins, that is, DPEP1, TFF1, ANPEP, LGALS3, PRG4, CDH17, PIGR, and TNFRSF10C, which exhibited high AUCs in the analysis results with the urinary Cr-relative values. The results are shown in Table 5 and Table 6.

**[Table 5]**

| Validation cohort (n = 111) | Univariate analysis | | | AUC [95% CI] | Multivariate analysis | |
|---|---|---|---|---|---|---|
| | HC Median [IQR] | CRC Median [IQR] | P value | | Odds ratio [95% CI] | P value |
| uDPEP1/uCr (ng/g.Cr) | 54.0 | 160.1 | < 0.001 | 0.809 | 1.010 | 0.003 |
| | [26.6 to 83.7] | [65.8 to 400.6] | | [0.730 to 0.888] | [1.003 to 1.016] | |
| uTFF1/uCr (ng/g.Cr) | 10.9 | 28.8 | < 0.001 | 0.731 | 1.012 | 0.115 |
| | [5.6 to 20.3] | [14.2 to 54.5] | | [0.634 to 0.828] | [0.997 to 1.028] | |
| uANPEP/uCr (ng/g.Cr) | 402.1 | 765.1 | < 0.001 | 0.741 | 1.001 | 0.435 |
| | [253.4 to 699.9] | [449.3 to 1002.6] | | [0.651 to 0.831] | [0.999 to 1.002] | |
| uLGALS3/uCr (ng/g.Cr) | 642.8 | 1179.5 | < 0.001 | 0.724 | 1.000 | 0.177 |
| | [359.1 to 1018.6] | [669.6 to 1622.1] | | [0.628 to 0.820] | [1.000 to 1.001] | |
| uPRG4/uCr (ng/g.Cr) | 105.5 | 124.4 | 0.146 | 0.581 | 1.000 | 0.875 |
| | [55.1 to 142.5] | [63.5 to 227.2] | | [0.469 to 0.692] | [0.998 to 1.002] | |
| uCDH17/uCr (ng/g.Cr) | 389.0 | 378.0 | 0.324 | 0.445 | 1.000 | 0.169 |
| | [249.0 to 718.8] | [176.3 to 610.2] | | [0.336 to 0.554] | [1.000 to 1.000] | |
| uPIGR/uCr (ng/g.Cr) | 157.9 | 176.3 | 0.282 | 0.560 | 1.001 | 0.765 |
| | [77.3 to 265.4] | [100.3 to 307.5] | | [0.452 to 0.667] | [0.997 to 1.004] | |
| uTNFRSF10C/uCr (µg/g.Cr) | 8.5 | 9.4 | 0.020 | 0.629 | 0.981 | 0.802 |
| | [6.9 to 10.0] | [7.8 to 12.4] | | [0.525 to 0.734] | [0.843 to 1.142] | |

**[Table 6]**

| Validation cohort (n = 111) | Univariate analysis | | | AUC [95% CI] | Multivariate analysis | |
|---|---|---|---|---|---|---|
| | HC Median [IQR] | CRC Median [IQR] | P value | | Odds ratio [95% CI] | P value |
| uDPEP1 (pg/ml) | 49.3 | 180.6 | < 0.001 | 0.930 | 1.044 | < 0.001 |
| | [34.2 to 80.0] | [136.3 to 352.9] | | [0.884 to 0.977] | [1.020 to 1.069] | |
| uTFF1 (ng/ml) | 1.2 | 4.4 | < 0.001 | 0.747 | 1.001 | 0.008 |
| | [0.6 to 2.5] | [1.6 to 6.4] | | [0.650 to 0.844] | [1.000 to 1.001] | |
| uANPEP (ng/ml) | 49.8 | 99.2 | < 0.001 | 0.846 | 1.058 | 0.004 |
| | [31.4 to 74.9] | [75.8 to 126.2] | | [0.771 to 0.921] | [1.018 to 1.110] | |
| uLGALS3 (pg/ml) | 679.1 | 1363.6 | < 0.001 | 0.716 | 1.000 | 0.488 |
| | [356.9 to 1250.9] | [707.9 to 4285.0] | | [0.618 to 0.814] | [0.999 to 1.001] | |
| uPRG4 (pg/ml) | 96.6 | 136.6 | 0.004 | 0.661 | 1.003 | 0.524 |
| | [85.6 to 130.9] | [84.8 to 237.0] | | [0.552 to 0.770] | [0.994 to 1.012] | |
| uCDH17 (pg/ml) | 435.9 | 435.9 | 0.903 | 0.493 | 0.999 | 0.039 |
| | [321.2 to 659.1] | [313.0 to 606.3] | | [0.385 to 0.601] | [0.997 to 1.000] | |
| uPIGR (ng/ml) | 19.0 | 24.9 | 0.029 | 0.621 | 1.000 | 0.221 |
| | [8.9 to 29.2] | [11.6 to 45.6] | | [0.514 to 0.727] | [1.000 to 1.000] | |
| uTNFRSF10C (ng/ml) | 9.7 | 15.2 | 0.003 | 0.667 | 0.911 | 0.357 |
| | [6.1 to 12.6] | [8.1 to 20.8] | | [0.560 to 0.774] | [0.747 to 1.111] | |

As shown in Table 5, in terms of the urinary Cr-relative values, 5 types of urinary proteins, that is, DPEP1, TFF1, ANPEP, LGALS3, and TNFRSF10C, also showed high expression in the urine of the colorectal cancer patients as compared with the healthy individuals in the validation cohort.

As shown in Table 6, in terms of the absolute values, the 7 types of urinary proteins other than CDH17, that is, DPEP1, TFF1, ANPEP, LGALS3, PRG4, PIGR, and TNFRSF10C, also showed high expression in the urine of the colorectal cancer patients as compared with the healthy individuals in the validation cohort.

These results are consistent with the discovery cohort and the training cohort. Furthermore, in the validation cohort, the AUC of urinary DPEP1 was the highest, with the urinary Cr-relative value being 0.809 (Table 5) and the absolute value being 0.930 (Table 6), as in the analysis in the training cohort. As described above, the urinary DPEP1 showed the highest reproducibility as a biomarker, enabling diagnosis of colorectal cancer with extremely high accuracy.

The diagnostic accuracy of urinary DPEP1 showed very high values in both cohorts, with AUC = 0.802 (Table 1) and AUC = 0.809 (Table 5).

As the next best candidate, the diagnostic accuracy of urinary TFF1 also showed high values in both cohorts, with AUC = 0.751 (Table 1) and AUC = 0.731 (Table 5).

### <Diagnostic ability of colorectal cancer biomarkers>

Table 7 shows an example of the cut-off values of various colorectal cancer biomarkers for colorectal cancer of stages 0 to III obtained from the analysis results with the urinary Cr-relative values, and the diagnostic accuracy.

Table 8 shows an example of the cut-off values of various colorectal cancer biomarkers for colorectal cancer of stages 0 to III obtained from the analysis results with the absolute values, and the diagnostic accuracy thereof.

The cut-off values (cut-off values) shown in the tables are merely values as an example applied in the current analysis. The cut-off values used in actual testing sites are not necessarily the numerical values shown as exemplary examples in the following tables. The same applies to Table 9 and Table 10 that will be described later.

In the present analysis, the cut-off values were calculated as follows. First, the values of (sensitivity, specificity, 1 - specificity) were calculated for each value of each factor, and an ROC curve was drawn. The Youden Index of the ROC curve was used as a cut-off value. The Youden Index is a point at which the value of (sensitivity + specificity - 1) is maximized. Logistic regression analysis was used for calculating the cut-off value for a combination of two or more factors. A formula for predicting colorectal cancer was created using the factors to be combined as explanatory variables, and predicted values were calculated. Thereafter, the sensitivity, the specificity, and 1 - specificity were calculated for each predicted value in the same manner, an ROC curve was drawn, and the Youden Index was used as the cut-off value.

However, the cut-off value used in an actual testing site is not necessarily limited to the Youden Index. The cut-off value can be set in consideration of the purpose of the colorectal cancer screening. For example, in some cases, it may be preferable to set the cut-off value while prioritizing the improvement of sensitivity.

**[Table 7]**

| Whole cohort Stage 0-III | Cut-off value | Sensitivity | Specificity | AUC |
|---|---|---|---|---|
| | | | | [95% CI] |
| uDPEP1/uCr (ng/g.Cr) | 64.0 | 80.4% | 64.5% | 0.804 |
| | | | | [0.758 to 0.850] |
| uDPEP1/uCr (ng/g.Cr) + uTFF1/uCr (ng/g.Cr) | 0.310 | 91.1% | 62.8% | 0.846 |
| | | | | [0.804 to 0.888] |
| uDPEP1/uCr (ng/g.Cr) + uANPEP/uCr (ng/g.Cr) | 0.374 | 82.3% | 64.5% | 0.809 |
| | | | | [0.763 to 0.855] |
| uDPEP1/uCr (ng/g.Cr) + uLGALS3/uCr (ng/g.Cr) | 0.359 | 83.4% | 64.0% | 0.824 |
| | | | | [0.780 to 0.868] |
| uDPEP1/uCr (ng/g.Cr) + uTFF1/uCr (ng/g.Cr) + uANPEP/uCr (ng/g.Cr) | 0.314 | 89.9% | 62.8% | 0.845 |
| | | | | [0.802 to 0.887] |
| uDPEP1/uCr (ng/g.Cr) + uTFF1/uCr (ng/g.Cr) + uLGALS3/uCr (ng/g.Cr) | 0.321 | 87.9% | 64.0% | 0.849 |
| | | | | [0.807 to 0.891] |
| uDPEP1/uCr (ng/g.Cr) + uANPEP/uCr (ng/g.Cr) + uLGALS3/uCr (ng/g.Cr) | 0.357 | 84.1% | 62.8% | 0.826 |
| | | | | [0.782 to 0.870] |
| uTFF1/uCr (ng/g.Cr) + uPRG4/uCr (ng/g.Cr) + uENPEP/uCr (ng/g.Cr) | 0.306 | 87.1% | 65.9% | 0.835 |
| | | | | [0.775 to 0.894] |
| uDPEP1/uCr (ng/g.Cr) + uTFF1/uCr (ng/g.Cr) + uPIGR/uCr (ng/g.Cr) | 0.317 | 85.4% | 62.2% | 0.837 |
| | | | | [0.793 to 0.880] |
| uDPEP1/uCr (ng/g.Cr) + uTFF1/uCr (ng/g.Cr) + uANPEP/uCr (ng/g.Cr) + uLGALS3/uCr (ng/g.Cr) | 0.327 | 86.6% | 64.0% | 0.849 |
| | | | | [0.808 to 0.891] |
| uDPEP1/uCr (ng/g.Cr) + uTFF1/uCr (ng/g.Cr) + uLGALS3/uCr (ng/g.Cr) + uCDH17/uCr (ng/g.Cr) | 0.312 | 87.3% | 64.2% | 0.854 |
| | | | | [0.814 to 0.895] |

**[Table 8]**

| Whole cohort Stage 0-III | Cut-off value | Sensitivity | Specificity | AUC |
|---|---|---|---|---|
| | | | | [95% CI] |
| uDPEP1 (pg/ml) | 70.1 | 85.4% | 64.5% | 0.846 |
| | | | | [0.805 to 0.888] |
| uDPEP1 (pg/ml) + uTFF1 (pg/ml) | 0.2931 | 94.3% | 63.4% | 0.870 |
| | | | | [0.833 to 0.908] |
| uDPEP1 (pg/ml) + uANPEP (ng/ml) | 0.324 | 84.2% | 64.5% | 0.852 |
| | | | | [0.811 to 0.893] |
| uDPEP1 (pg/ml) + uLGALS3 (pg/ml) | 0.314 | 87.9% | 62.2% | 0.846 |
| | | | | [0.805 to 0.887] |
| uDPEP1 (pg/ml) + uPRG4 (pg/ml) | 0.307 | 87.3% | 61.0% | 0.857 |
| | | | | [0.817 to 0.897] |
| uDPEP1 (pg/ml) + uTFF1 (pg/ml) + uANPEP (ng/ml) | 0.311 | 85.4% | 65.1% | 0.862 |
| | | | | [0.823 to 0.902] |
| uDPEP1 (pg/ml) + uTFF1 (pg/ml) + uLGALS3 (pg/ml) | 0.310 | 90.4% | 66.9% | 0.870 |
| | | | | [0.833 to 0.908] |
| uDPEP1 (pg/ml) + uTFF1 (pg/ml) + uPRG4 (pg/ml) | 0.2833 | 92.4% | 62.8% | 0.876 |
| | | | | [0.840 to 0.912] |
| uDPEP1 (pg/ml) + uTFF1 (pg/ml) + uTNFRSF10C (ng/ml) | 0.293 | 94.9% | 64.5% | 0.882 |
| | | | | [0.847 to 0.918] |
| uDPEP1 (pg/ml) + uANPEP (ng/ml) + uLGALS3 (pg/ml) | 0.323 | 84.1% | 64.5% | 0.851 |
| | | | | [0.810 to 0.893] |
| uDPEP1 (pg/ml) + uTFF1 (pg/ml) + uPIGR (pg/ml) | 0.290 | 94.3% | 62.8% | 0.870 |
| | | | | [0.832 to 0.907] |
| uDPEP1 (pg/ml) + uTFF1 (pg/ml) + uANPEP (ng/ml) + uLGALS3 (pg/ml) | 0.309 | 87.3% | 66.3% | 0.865 |
| | | | | [0.826 to 0.904] |
| uDPEP1 (pg/ml) + uTFF1 (pg/ml) + uPRG4 (pg/ml) + uTNFRSF10C (ng/ml) | 0.284 | 93.6% | 64.5% | 0.889 |
| | | | | [0.855 to 0.923] |
| uDPEP1 (pg/ml) + uTFF1 (pg/ml) + uANPEP (ng/ml) + uPRG4 (pg/ml) + uTNFRSF10C (ng/ml) | 0.269 | 92.4% | 63.4% | 0.889 |
| | | | | [0.855 to 0.923] |

As shown in Table 7 and Table 8, in the case of the urinary DPEP1 alone, the sensitivity was 80.4% while the specificity was 64.5% in terms of the urinary Cr-relative value, and the sensitivity was 85.4% while the specificity was 64.5% in terms of the absolute value. When the urinary DPEP1 is used, it is believed that colorectal cancer can be determined with exceptional sensitivity and specificity.

Furthermore, the 16 types of urinary proteins can also be utilized for diagnosis even when two or more kinds are combined. For example, various combination patterns centered on the DPEP1 or TFF1 may be considered. Examples of the combination pattern and the sensitivity and specificity thereof are shown together in Table 7 and Table 8.

When a combination centered on two types of proteins, urinary DPEP1 and urinary TFF1, which have reproducibility under all conditions, was used, the AUC had high values of 0.846 in terms of the urinary Cr-relative value and 0.870 in terms of the absolute value. In addition, the combination showed high diagnostic accuracy, with a sensitivity of 91.1% and a specificity of 62.8% in terms of the urinary Cr-relative value, and a sensitivity of 94.3% and a specificity of 63.4% in terms of the absolute value.

### <Diagnostic ability for early colorectal cancer>

Table 9 shows an example of the cut-off values of various colorectal cancer biomarkers for colorectal cancer of stage 0/I obtained from the analysis results with the urinary Cr-relative values, and the diagnostic accuracy.

Table 10 shows an example of the cut-off values of various colorectal cancer biomarkers for colorectal cancer of stage 0/I obtained from the analysis results with the absolute values, and the diagnostic accuracy.

The method for calculating the cut-off values shown in each of Table 9 and Table 10 is the same as the method for calculating the cut-off values shown in each of Table 7 and Table 8. That is, the Youden Index of the ROC curve was used as the cut-off value. Furthermore, logistic regression analysis was used for calculating the cut-off value for a combination of two or more factors.

However, the cut-off value used in an actual testing site is not necessarily limited to the values shown in Table 9 and Table 10. The cut-off value can be set in consideration of the purpose of the colorectal cancer screening. For example, in some cases, it may be preferable to set the cut-off value while prioritizing the improvement of sensitivity.

**[Table 9]**

| Whole cohort Stage O/I | Cut-off value | Sensitivity | Specificity | AUC |
|---|---|---|---|---|
| | | | | [95% CI] |
| uDPEP1/uCr (ng/g.Cr) | 64.0 | 80.0% | 64.5% | 0.780 |
| | | | | [0.715 to 0.845] |
| uDPEP1/uCr (ng/g.Cr) + uTFF1/uCr (ng/g.Cr) | 0.212 | 81.5% | 66.3% | 0.792 |
| | | | | [0.727 to 0.857] |
| uDPEP1/uCr (ng/g.Cr) + uANPEP/uCr (ng/g.Cr) | 0.225 | 78.5% | 70.3% | 0.777 |
| | | | | [0.711 to 0.843] |
| uDPEP1/uCr (ng/g.Cr) + uLGALS3/uCr (ng/g.Cr) | 0.208 | 87.5% | 61.6% | 0.807 |
| | | | | [0.746 to 0.867] |
| uDPEP1/uCr (ng/g.Cr) + uTFF1/uCr (ng/g.Cr) + uANPEP/uCr (ng/g.Cr) | 0.2005 | 86.2% | 64.0% | 0.797 |
| | | | | [0.734 to 0.860] |
| uDPEP1/uCr (ng/g.Cr) + uTFF1/uCr (ng/g.Cr) + uLGALS3/uCr (ng/g.Cr) | 0.2121 | 82.8% | 64.5% | 0.803 |
| | | | | [0.740 to 0.866] |
| uDPEP1/uCr (ng/g.Cr) + uANPEP/uCr (ng/g.Cr) + uLGALS3/uCr (ng/g.Cr) | 0.214 | 81.3% | 65.7% | 0.803 |
| | | | | [0.742 to 0.864] |
| uTFF1/uCr (ng/g.Cr) + uPRG4/uCr (ng/g.Cr) + uENPEP/uCr (ng/g.Cr) | 0.197 | 81.3% | 63.5% | 0.765 |
| | | | | [0.674 to 0.856] |
| uDPEP1/uCr (ng/g.Cr) + uTFF1/uCr (ng/g.Cr) + uPIGR/uCr (ng/g.Cr) | 0.209 | 80.0% | 61.6% | 0.774 |
| | | | | [0.703 to 0.844] |
| uDPEP1/uCr (ng/g.Cr) + uTFF1/uCr (ng/g.Cr) + uANPEP/uCr (ng/g.Cr) + uLGALS3/uCr (ng/g.Cr) | 0.201 | 84.4% | 61.6% | 0.806 |
| | | | | [0.744 to 0.868] |
| uDPEP1/uCr (ng/g.Cr) + uTFF1/uCr (ng/g.Cr) + uLGALS3/uCr (ng/g.Cr) + uCDH17/uCr (ng/g.Cr) | 0.2058 | 81.3% | 65.1% | 0.799 |
| | | | | [0.735 to 0.863] |

**[Table 10]**

| Whole cohort Stage 0/I | Cut-off value | Sensitivity | Specificity | AUC |
|---|---|---|---|---|
| | | | | [95% CI] |
| uDPEP1 (pg/ml) | 70.1 | 84.6% | 66.9% | 0.842 |
| | | | | [0.788 to 0.896] |
| uDPEP1 (pg/ml) + uTFF1 (pg/ml) | 0.1695 | 93.8% | 61.6% | 0.852 |
| | | | | [0.801 to 0.902] |
| uDPEP1 (pg/ml) + uANPEP (ng/ml) | 0.193 | 83.1% | 65.7% | 0.832 |
| | | | | [0.773 to 0.891] |
| uDPEP1 (pg/ml) + uLGALS3 (pg/ml) | 0.182 | 84.4% | 61.6% | 0.836 |
| | | | | [0.783 to 0.890] |
| uDPEP1 (pg/ml) + uPRG4 (pg/ml) | 0.185 | 86.2% | 63.4% | 0.838 |
| | | | | [0.782 to 0.894] |
| uDPEP1 (pg/ml) + uTFF1 (pg/ml) + uANPEP (ng/ml) | 0.174 | 86.2% | 60.5% | 0.835 |
| | | | | [0.775 to 0.895] |
| uDPEP1 (pg/ml) + uTFF1 (pg/ml) + uLGALS3 (pg/ml) | 0.1813 | 87.5% | 63.4% | 0.846 |
| | | | | [0.794 to 0.898] |
| uDPEP1 (pg/ml) + uTFF1 (pg/ml) + uPRG4 (pg/ml) | 0.172 | 89.2% | 63.4% | 0.850 |
| | | | | [0.799 to 0.901] |
| uDPEP1 (pg/ml) + uTFF1 (pg/ml) + uTNFRSF10C (ng/ml) | 0.1794 | 95.3% | 65.1% | 0.862 |
| | | | | [0.814 to 0.910] |
| uDPEP1 (pg/ml) + uANPEP (ng/ml) + uLGALS3 (pg/ml) | 0.181 | 85.9% | 63.4% | 0.828 |
| | | | | [0.768 to 0.888] |
| uDPEP1 (pg/ml) + uTFF1 (pg/ml) + uPIGR (pg/ml) | 0.175 | 89.2% | 63.4% | 0.847 |
| | | | | [0.796 to 0.899] |
| uDPEP1 (pg/ml) + uTFF1 (pg/ml) + uANPEP (ng/ml) + uLGALS3 (pg/ml) | 0.172 | 89.1% | 60.5% | 0.834 |
| | | | | [0.774 to 0.894] |
| uDPEP1 (pg/ml) + uTFF1 (pg/ml) + uPRG4(pg/ml) + uTNFRSF 10C (ng/ml) | 0.175 | 89.1% | 62.2% | 0.844 |
| | | | | [0.791 to 0.897] |
| uDPEP1 (pg/ml) + uTFF1 (pg/ml) + uANPEP (ng/ml) + uPRG4 (pg/ml) + uTNFRSF10C (ng/ml) | 0.169 | 87.5% | 61.0% | 0.851 |
| | | | | [0.797 to 0.905] |

When the established colorectal cancer biomarkers are used, early colorectal cancer of stage 0/I that can be resected by endoscopic treatment can also be diagnosed with high accuracy.

As shown in FIG. 4 to FIG. 11, the expression levels of four urinary proteins (DPEP1, TFF1, ANPEP, and LGALS3) in each stage, which show exceptional sensitivity and specificity when used alone, showed higher values from the stage 0 as compared with healthy individuals for all the proteins. In addition, the expression levels of all the proteins maintained high values in stages I to III as compared with healthy individuals. Moreover, all the proteins show a graph tendency suitable for the diagnosis of early colorectal cancer.

In the case of DPEP1 alone, the AUC for the diagnosis of early colorectal cancer was such that AUC = 0.780 in terms of the urinary Cr-relative value and AUC = 0.842 in terms of the absolute value. In the case of the combination of DPEP1 + TFF1, the AUC was such that AUC = 0.792 in terms of the urinary Cr-relative value, and AUC = 0.852 in terms of the absolute value (Table 9 and Table 10).

### <Diagnostic ability for adenoma>

The concentration of each of DPEP1 and TFF1 in the urine specimens collected from 172 cases of healthy individuals, 159 cases of colorectal cancer patients, and 31 cases of patients with adenoma were measured. The results are shown in FIG. 12 to FIG. 19, and Table 11 and Table 12. Table 11 shows the urinary Cr-relative values each standardized by dividing the urinary protein concentration by the urinary creatinine concentration. Table 12 shows the absolute value of each urinary protein concentration.

**[Table 11]**

| Whole cohort | Univariate analysis | | | P value | | |
|---|---|---|---|---|---|---|
| | HC | Adenoma | CRC | HC vs. Adenoma | HC vs. CRC | Adenoma vs. CRC |
| | Median [IQR] | Median [IQR] | Median [IQR] | | | |
| uDPEP1/uCr (ng/g.Cr) | 50.5 | 57.4 | 123.2 | 0.037 | < 0.001 | 0.001 |
| | [28.9 to 80.0] | [40.0 to 128.3] | [73.9 to 228.4] | | | |
| uTFF1/uCr (ng/g.Cr) | 12.1 | 15.9 | 31.9 | 0.001 | < 0.001 | 0.041 |
| | [6.0 to 19.7] | [12.9 to 37.6] | [16.1 to 56.6] | | | |

**[Table 12]**

| Whole cohort | Univariate analysis | | | P value | | |
|---|---|---|---|---|---|---|
| | HC | Adenoma | CRC | HC vs. Adenoma | HC vs. CRC | Adenoma vs. CRC |
| | Median [IQR] | Median [IQR] | Median [IQR] | | | |
| uDPEP1 (pg/ml) | 57.4 | 77.9 | 141.5 | 0.013 | < 0.001 | < 0.001 |
| | [37.9 to 81.0] | [48.8 to 118.2] | [83.4 to 215.0] | | | |
| uTFF1 (ng/ml) | 1.3 | 2 | 3.7 | 0.003 | < 0.001 | 0.034 |
| | [0.7 to 2.6] | [1.6 to 3.6] | [1.5 to 7.0] | | | |

It was found that DPEP1 and TFF1 in the urine specimens were significantly highly expressed in the patients with adenoma than in the healthy individuals. These results suggest the usefulness in the detection of adenoma in addition to early colorectal cancer.

Next, an ROC curve for making a decision on the presence or absence of the contraction of adenoma was created from the measurement results of each urinary protein concentration (FIG. 20 and FIG. 21). FIG. 20 shows an ROC curve based on the urinary Cr-relative value standardized by dividing each urinary protein concentration by the urinary creatinine concentration, and FIG. 21 shows an ROC curve based on the absolute value of each urinary protein concentration.

In the case of the ROC curve of the urinary Cr-relative value in FIG. 20, the AUC of DPEP1 was 0.618, and the AUC of TFF1 was 0.684. In the case of the ROC curve of the absolute value in FIG. 21, the AUC of DPEP1 was 0.641, and the AUC of TFF1 was 0.668.

### <Investigation on serum specimen>

The concentration of each of DPEP1 and TFF1 in the serum specimen collected from each of 68 cases of healthy individuals and 68 cases of colorectal cancer patients was measured. The results are shown in Table 13. Table 13 shows the absolute value of each urinary protein concentration. FIG. 22 shows an ROC curve drawn based on the measured values of each protein concentration. The AUC value was acquired from this ROC curve.

**[Table 13]**

| serum cohort (n = 136) | Univariate analysis | | P value | AUC [95% CI] |
|---|---|---|---|---|
| | HC | CRC | | |
| | Median [IQR] | Median [IQR] | | |
| sDPEP1 (pg/ml) | 182 | 221.2 | 0.023 | 0.613 |
| | [104.7 to 290.3] | [124.1 to 487.4] | | [0.519 to 0.707] |
| sTFF1 (pg/ml) | 78.5 | 161.6 | < 0.001 | 0.709 |
| | [54.9 to 126.8] | [86.9 to 225.6] | | [0.621 to 0.796] |

From the above results, it is considered that a novel and revolutionary colorectal cancer biomarker that enables diagnosis of even early colorectal cancer with high accuracy and in a non-invasive manner, can be provided. It is difficult to realize diagnosing early colorectal cancer with high accuracy and in a non-invasive manner, when existing fecal occult blood or serum tumor markers are used. It is expected that use of the colorectal cancer biomarker of the present invention makes it possible to provide not only a test kit used in a facility but also a simple kit that can be used for testing at home.

### INDUSTRIAL APPLICABILITY

According to the present invention, there is provided a colorectal cancer biomarker that enables simple and non-invasive determination of the presence or absence of the contraction of colorectal cancer with exceptional sensitivity and specificity.

## Claims

1. A method for testing for colorectal cancer, the method comprising:
detecting at least one urinary protein selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.

2. A method for diagnosing colorectal cancer, the method comprising:
detecting at least one urinary protein selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.

3. The method according to Claim 1 or 2, further comprising:
determining that, in a case where the urinary protein is at least one selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, and MME, a subject has contracted colorectal cancer when a concentration of the detected urinary protein is equal to or greater than a cut-off value; and
determining that, in a case where the urinary protein is ACP2, a subject has contracted colorectal cancer when a concentration of the detected urinary protein is equal to or less than a cut-off value.

4. The method according to any one of Claims 1 to 3,
wherein the urinary protein is at least one selected from the group consisting of DPEP1, TFF1, ANPEP, LGALS3, PRG4, PIGR, and TNFRSF10C.

5. The method according to any one of Claims 1 to 4,
wherein the urinary protein is a combination of DPEP1 and at least one selected from the group consisting of TFF1, ANPEP, LGALS3, PRG4, PIGR, and TNFRSF10C.

6. The method according to any one of Claims 1 to 5,
wherein the urinary protein is a combination of TFF1 and at least one selected from the group consisting of DPEP1, ANPEP, LGALS3, PRG4, PIGR, and TNFRSF10C.

7. The method according to any one of Claims 1 to 6,
wherein the urinary protein is at least one selected from the group consisting of DPEP1, TFF1, ANPEP, and LGALS3.

8. The method according to any one of Claims 1 to 7,
wherein the colorectal cancer is early colorectal cancer.

9. A method for treating colorectal cancer, the method comprising:
diagnosing a subject using the method according to any one of Claims 1 to 8; and
administering a therapeutic drug for colorectal cancer to the subject when the subject is diagnosed as having contracted colorectal cancer.

10. A method for treating colorectal cancer, the method comprising:
diagnosing a subject using the method according to any one of Claims 1 to 8; and
performing surgery on the subject when the subject is diagnosed as having contracted colorectal cancer.

11. A colorectal cancer test kit used for a test of colorectal cancer, the kit comprising:
a container for collecting urine of a subject; and
a reagent for detecting at least one urinary protein selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.

12. The colorectal cancer test kit according to Claim 11,
wherein the reagent is either or both of the following reagent α and reagent β,
reagent α: a reagent for notifying, by a qualitative technique, that in a case where the urinary protein is at least one selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, and MME, the subject has contracted colorectal cancer when the concentration of the detected urinary protein is equal to or greater than a threshold value; and
a reagent β: a reagent for notifying, by a qualitative technique, that in a case where the urinary protein is ACP2, the subject has contracted colorectal cancer when the concentration of the detected urinary protein is equal to or less than a threshold value.

13. The colorectal cancer test kit according to Claim 11 or 12,
wherein the urinary protein is at least one selected from the group consisting of DPEP1, TFF1, ANPEP, LGALS3, PRG4, PIGR, and TNFRSF10C.

14. The colorectal cancer test kit according to any one of Claims 11 to 13,
wherein the urinary protein is a combination of DPEP1 and at least one selected from the group consisting of TFF1, ANPEP, LGALS3, PRG4, PIGR, and TNFRSF10C.

15. The colorectal cancer test kit according to any one of Claims 11 to 14,
wherein the urinary protein is a combination of TFF1 and at least one selected from the group consisting of DPEP1, ANPEP, LGALS3, PRG4, PIGR and TNFRSF10C.

16. The colorectal cancer test kit according to any one of Claims 11 to 15,
wherein the urinary protein is at least one selected from the group consisting of DPEP1, TFF1, ANPEP, and LGALS3.

17. The colorectal cancer test kit according to any one of Claims 11 to 16,
wherein the colorectal cancer is early colorectal cancer.

18. Use of a urinary protein for testing and/or diagnosis of colorectal cancer,
wherein the urinary protein is at least one selected from the group consisting of DPEP1, TFF1, ANPEP, LGALS3, PRG4, CDH17, PIGR, TNFRSF10C, MEP1A, LGALS3BP, APOA1, LRG1, ENPEP, S100A11, MME, and ACP2.

19. The use according to Claim 18,
wherein the urinary protein is at least one selected from the group consisting of DPEP1, TFF1, ANPEP, LGALS3, PRG4, PIGR, and TNFRSF10C.

20. The use according to Claim 18 or 19,
wherein the urinary protein is a combination of DPEP1 and at least one selected from the group consisting of TFF1, ANPEP, LGALS3, PRG4, PIGR, and TNFRSF10C.

21. The use according to any one of Claims 18 to 20,
wherein the urinary protein is a combination of TFF1 and at least one selected from the group consisting of DPEP1, ANPEP, LGALS3, PRG4, PIGR, and TNFRSF10C.

22. The use according to any one of Claims 18 to 21,
wherein the urinary protein is at least one selected from the group consisting of DPEP1, TFF1, ANPEP, and LGALS3.

23. The use according to any one of Claims 18 to 22,
wherein the colorectal cancer is early colorectal cancer.
